(19) Europäisches Patentamt
European Patent Office
Office européen des brevets

(11) Veröffentlichungsnummer: **0 110 832**
**B1**

(12) **EUROPÄISCHE PATENTSCHRIFT**

(45) Veröffentlichungstag der Patentschrift: **27.12.89**

(21) Anmeldenummer: **83810541.9**

(22) Anmeldetag: **21.11.83**

(51) Int. Cl.⁴: **C 07 D 311/86,** C 07 D 335/16, C 07 C 121/75, C 07 C 149/40, C 07 D 209/48

(54) **Xanthone und Thioxanthone.**

(30) Priorität: **25.11.82 CH 6872/82**

(43) Veröffentlichungstag der Anmeldung: **13.06.84 Patentblatt 84/24**

(45) Bekanntmachung des Hinweises auf die Patenterteilung: **27.12.89 Patentblatt 89/52**

(84) Benannte Vertragsstaaten: **CH DE FR GB LI NL**

(73) Patentinhaber: **CIBA- GEIGY AG, Klybeckstrasse 141, CH- 4002 Basel (CH)**

(72) Erfinder: **Fischer, Walter, Dr., Vogesenstrasse 77, CH- 4153 Reinach (CH)**
Erfinder: **Finter, Jürgen, Dr., Zasiusstrasse 100, D-7800 Freiburg (DE)**
Erfinder: **Zweifel, Hans, Dr., Lothringerstrasse 93, CH- 4056 Basel (CH)**

(56) Entgegenhaltungen:
**EP-A-0 033 720**
**EP-A-0 062 615**
**CH-A-580 093**
**DD-A-106 263**
**DE-A-2 120 708**
**FR-A-2 482 098**
**GB-A-2 067 998**
**JP-A-7 131 731**
**US-A-3 534 063**
**US-A-3 922 284**
**US-A-4 011 246**
**US-A-4 250 182**
**US-A-4 257 953**
**US-A-4 363 917**

**CHEMICAL ABSTRACTS, Vol. 92, No. 13, 31 März 1980, Columbus, Ohio, USA ABDEL-MEGEID, F.M.E. ET AL., "Some reactions of 4-chronome**

(56) Entgegenhaltungen: (Fortsetzung)
**derivatives" Seite 693, Spalte 1, Abstract No. 110 778s**
**CHEMICAL ABSTRACTS, Vol. 77, No. 1, 3. Juli 1972, Columbus, Ohio, USA Arunachalam T. ET AL.,."Synthesis of a hydroxyxanthone dicarboxylic acid, cassiaxanthone.Reactions of alpha-resorcylic acid with phenols" Seite 465, Spalte 2, Abstract No. 5 281s**
**CHEMICAL ABSTRACTS, Vol. 89, No. 5, 31. Juli 1978, Columbus, Ohio, USA KATO:"Chemical reactions of bikaverin" Seite 593, Spalte 1, Abstract No. 43 012k**
**CHEMICAL ABSTRACTS, Vol. 87, No. 4, 25. Juli 1977, Columbus, Ohio, USA JONES WINTON D. ET AL., "Antiallergic agents.Xanthone-2,7-dicarboxylic acid derivatives" Seite 8, Spalte 2, Abstract No. 33 420m**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

EP 0 110 832 B1

**Beschreibung**

FR-A-2 482 098 offenbart 3-(2-Carboxy-phenylthio)phthalsäureimide als Photosensibilisatoren. Thioxanthoncarbonsäurederivate und deren Verwendung als Sensibilisatoren für lichtvernetzbare Polymere oder als Photoinitiatoren sind aus EP-A-33 720 bekannt. EP-A-62 615 offenbart tetrasubstituierte Phthalsäurederivate mit ähnlichen Ersatzmöglichkeiten. Chem. Abs. *77* (1972), 5281s offenbart Hydroxyxanthone, ohne dass eine Verwendungsmöglichkeit angegeben wird. Die JP-7 131 731 (Sho 46 - 31 731) betrifft Polymerzusammensetzungen, welche auch Xanthone und Thioxanthone umfassen, über deren photosensibilisierende Wirkung keine Angaben gemacht werden. Die Massgabe des Hauptanspruchs der vorliegenden Erfindung bezieht sich auf Verbindungen, die aus Sho 46 - 31 731 bekannt sind.

Die vorliegende Erfindung betrifft neue Xanthone und Thioxanthone sowie Verfahren zu deren Herstellung; die neuen Xanthone und Thioxanthone können als Photosensibilisatoren und im Gemisch mit Polymeren, die H-Donorgruppen enthalten, zur stromlosen Metallabscheidung verwendet werden.

Gegenstand vorliegender Erfindung sind Xanthone und Thioxanthone der Formel I

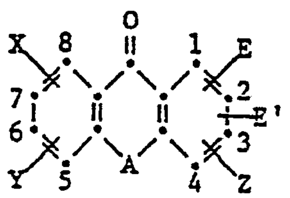

(I)

worin

A -O- oder -S-,
X und Y unabhängig voneinander Wasserstoff, $C_{1-20}$-Alkyl, Halogen, -OR', -SR' oder $-NO_2$'
Z Wasserstoff, $C_{1-14}$-Alkyl, Halogen, -OR', -SR', $-NO_2$, $-NH_2$' -OH oder $-NHCOCH_3$'
E und E' unabhängig voneinander -COOR", $-CON(R")_2$ oder -CN oder zusammen -CO-O-CO- oder -CO-N(R)-CO-,
R Wasserstoff, $C_{1-20}$-Alkyl, Phenyl, Alkylphenyl mit 1 - 4 C-Atomen im Alkyl, Phenyläthyl, Benzyl, Cyclohexyl, $-(CH_2)_n$-Q, $C_{2-6}$-Alkenyl, Propargyl, $-N(CH_3)_2$, -OH oder $C_{1-10}$-Alkoxy,
R' $C_{1-20}$-Alkyl, Phenyl, Halogenphenyl, Nitrophenyl, Alkyl- oder Alkoxyphenyl mit je 1 - 4 C-Atomen im Alkyl oder Alkoxy, Benzyl oder Phenyläthyl,
R" die unabhängig voneinander Wasserstoff, $M^+$, $C_{1-20}$-Alkyl, Alkoxyalkoxyalkyl mit 3 - 10 Atomen oder Hydroxyalkyl mit 2 - 8 Atomen,
n die Zahl 1, 2 oder 3,
Q -OH, $-N(C_{1-3}\text{-Alkyl})_2$, $-SO_3^-M^+$, $-OCOCH{=}CH_2$ oder $-OCOC(CH_3{=}CH_2$ und
$M^+$ ein Alkalimetallkation darstellen,

wobei, wenn A -O- darstellt, E und E' zusammen -CO-O-CO- oder -CO-N(R)-CO- bedeuten.

Stellen X, Y, R, R' und R" Alkylgruppen mit 1 - 20 C-Atomen dar, so kann es sich dabei um geradkettige oder verzweigte Gruppen handeln, wie z. B. Methyl, Äthyl, n-Propyl, Isopropyl, n-Butyl, sek.-Butyl, tert.-Butyl, n-Pentyl, 2- oder 3-Pentyl, n-Hexyl, 2-Ähylhexyl, n-Heptyl, 2- oder 3-Heptyl, n-Octyl, 1,1,3,3-Tetramethylbutyl, n-Nonyl, n-Decyl, 2-Decyl, n-Dodecyl, n-Tetradecyl, n-Hexadecyl, n-Octadecyl, Tridec-7-yl, Heptadec-9-yl, 2,6,10-Trimethyldodecyl und 2,6,10,14-Tetramethylhexadecyl. Bevorzugt weisen Alkylgruppen X, Y, R, R' oder R" 1 - 10 C-Atome auf. Alkoxygruppen R können ebenfalls geradkettig oder verzweigt sein, wie die Methoxy-, Äthoxy-, n-Propoxy-, Isopropoxy-, n-Butyloxy-, sek-Butyloxy-, n-Pentyloxy-, n-Hexyloxy-, 2-Äthylhexyloxy-, n-Octyloxy- und n-Decyloxygruppe. Bevorzugt sind geradkettige Alkoxygruppen R mit 1 - 4 C-Atomen.

Bedeuten X, Y oder Z Halogenatome oder sind solche Reste durch Halogenatome substituiert, so handelt es sich dabei z. B. um Fluor- und insbesondere um Brom- oder Chloratome.

Z in der Bedeutung von $C_{1-4}$-Alkyl ist beispielsweise Methyl, Äthyl, n-Propyl, Isopropyl oder n-Butyl, besonders Äthyl und vor allem Methyl.

Als Alkylphenylgruppen R oder R' und Alkoxyphenylgruppen R kommen vor allem derartige Gruppen mit 1 - 2 C-Atomen im Alkyl oder Alkoxy in Betracht, wie Methylphenyl, Äthylphenyl, Methoxy- und Äthoxyphenyl.

Stellt R Phenyläthyl dar, so handelt es sich vor allem um die 2-Phenyläthylgruppe. Bedeutet R $C_{2-6}$-Alkenyl, so kommen z. B. die Vinyl-, Allyl-, Methallyl-, Buten-1-yl- und Hexen-1-ylgruppe in Betracht.

R" in der Bedeutung einer Alkoxyalkoxyalkyl- oder Hydroxyalkylgruppe ist z. B. Methoxymethoxymethyl, Methoxy-2-methoxyäthyl,2-(2-Methoxyäthoxy)-äthyl, 2-(2-Äthoxyäthoxy)-äthyl, 2-(3-Propoxyäthoxy)-äthyl oder 2-(4-Butoxyäthoxy)-äthyl; 2-Hydroxyäthyl, 2- oder 3-Hydroxypropyl, 4-Hydroxybutyl, 3-Hydroxybutyl, 5-Hydroxypentyl, 2-Hydroxyhexyl und 8-Hydroxyoctyl. Alkoxyalkoxyalkylgruppen R" weisen bevorzugt 5-10 C-Atome auf; besonders bevorzugt sind die 2-(2-Methoxyäthoxy)-äthyl- und 2-(2-Äthoxyäthoxy)-äthylgruppen.

Als Alkalimetallkationen M kommen insbesondere das Kalium- und Natriumkation in Betracht.

A ist vorzugsweise -S-; X und Y stellen bevorzugt unabhängig voneinander Wasserstoff, Chlor, Brom, $C_{1-4}$-Alkyl oder $-NO_2$ dar. Besonders bevorzugt sind X und Y je Wasserstoff.

Bevorzugte Bedeutungen von Z sind Wasserstoff, $C_{1-4}$-Alkyl, Chlor, Brom, $C_{1-10}$-Alkoxy, $C_{1-10}$-Alkylthio, Phe-

nylthio, $-NO_2$, $-NH_2$ oder -OH.

R stellt bevorzugt Wasserstoff, $C_{1-10}$-Alkyl, Phenyl, Tolyl, Benzyl, $-(CH_2)_n$ -Q mit n = 2 oder 3 und Q = -OH, $-SO_3^-Na^+$, $-N(CH_3)_2$, $-N(C_2H_5)_2$, $-OCOCH=CH_2$ oder $-OCOC(CH_3)=CH_2$; $C_{2-6}$-Alkenyl, besonders Allyl, Propargyl oder $-N(CH_3)_2$ dar.

Bevorzugt sind Verbindungen der Formel I, worin X und Y Wasserstoff und A -S- darstellen und Halogenatome Z oder Halogensubstituenten in Z Fluor, Chlor oder Brom, besonders Chlor oder Brom, sind.

E und E' sind vorzugsweise in Nachbarstellung zueinander oder in 1, 3-Stellung gebunden. Bevorzugt sind derartige Verbindungen, worin X und Y Wasserstoff, A -S- und Z Wasserstoff, Chlor, $-NO_2$, $-NH_2$, -OH, -OR' oder -SR' darstellen, E und E' in Nachbarstellung zueinander gebunden sind und unabhängig voneinander eine Gruppe -COOR" oder zusammen -CO-O-CO- oder -CO-N(R)-CO bedeuten, R die unter Formel I angegebene Bedeutung hat, R' $C_{1-10}$-Alkyl, Benzyl, Phenyl oder Tolyl, die Reste R" unabhängig voneinander Wasserstoff, $M^+$, $C_{1-10}$-Alkyl oder Alkoxyalkoxyalkyl mit 5 - 10 C-Atomen bedeuten.

Besonders bevorzugt sind Verbindungen der Formel Ia

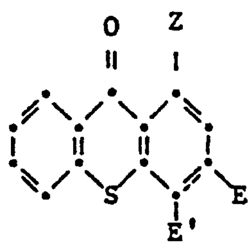

(Ia)

worin Z Wasserstoff, Chlor, $-NO_2$, $-NH_2$, -OH oder -SR', R' $C_{1-10}$-Alkyl oder Phenyl, E und E' unabhängig voneinander -COOR" oder zusammen -CO-O-CO- oder -CO-N(R)-CO-, die R" unabhängig voneinander Wasserstoff, $M^+$, $C_{1-10}$-Alkyl oder Alkoxyalkoxyalkyl mit 5 - 10 C-Atomen bedeuten und R die unter Formel I angegebene Bedeutung hat, vor allem Verbindungen der Formel Ia, worin Z Wasserstoff, E und E' unabhängig voneinander -COOR" oder zusammen -CO-O-CO- oder -CO-N(R)-CO-, R Wasserstoff, $C_{1-10}$-Alkyl, Phenyl, Tolyl, Benzyl, $-(CH_2)_n-OH$, $-(CH_2)_n-N(C_{1-10}\text{-Alkyl})_2$ mit n = 2 oder 3, $-CH_2CH_2OCOC(CH_3)=CH_2$, Allyl oder Propargyl und die R" unabhängig voneinander Wasserstoff, $Na^+$, $C_{1-10}$-Alkyl oder $C_{1-2}$-Alkoxy-$C_{1-3}$-Alkoxy-$_{1-2}$-Alkyl bedeuten. Ganz besonders bevorzugt ist die Verbindung der Formel Ia, worin Z Wasserstoff ist und E und E' zusammen $-CO-N(CH_2CH=CH_2)-CO-$ bedeuten.

Die Verbindungen der Formel I können z. B. dadurch hergestellt werden, dass man,

a) wenn E und E' in Nachbarstellung zueinander gebunden sind, eine Verbindung der Formel II

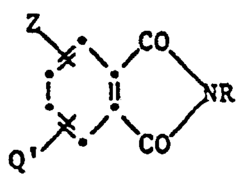

(II)

in Gegenwart einer Base mit einer Verbindung der Formel III

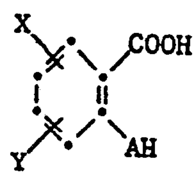

(III)

oder mit einem Salz davon zu einer Verbindung der Formel IV

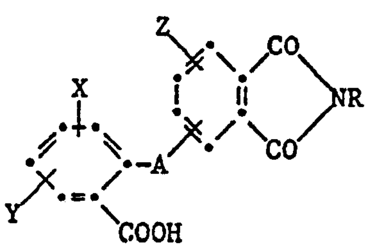

(IV)

umsetzt, die Verbindung der Formel IV, gegebenenfalls unter vorheriger Überführung in das Säurechlorid, zu einer Verbindung der Formel Ib

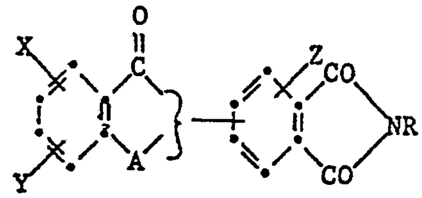

(Ib)

cyclisiert und diese gegebenenfalls anschliessend in eine Verbindung der Formel I überführt, worin E und E' unabhängig voneinander -COOR", $-CON(R")_2$, -CN oder zusammen -CO-O-CO- oder -CO-N(R)-CO- mit unterschiedlicher Bedeutung von R darstellen, oder

b) zur Herstellung von Verbindungen der Formel I, worin E und E' unabhängig voneinander -COOR", $-CON(R")_2$ oder -CN darstellen, eine Verbindung der Formel IIa

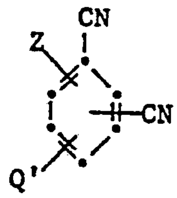

(IIa)

in Gegenwart einer Base mit einer Verbindung der Formel III oder mit einem Salz einer Verbindung der Formel III zu einer Verbindung der Formel IVa

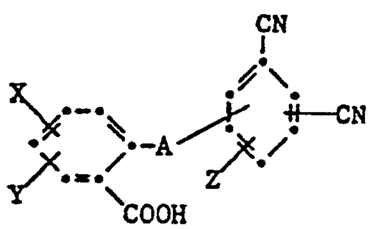

(IVa)

umsetzt, die Verbindung der Formel IVa zu einer Verbindung der Formel Ic

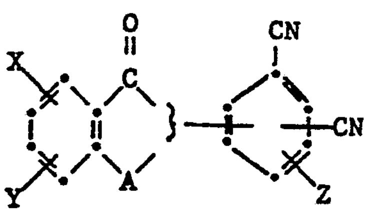

(Ic)

cyclisiert und diese gegebenenfalls anschliessend in eine Verbindung der Formel I überführt, worin E und E' unabhängig voneinander -COOR" oder $-CON(R")_2$ darstellen,

c) oder eine Verbindung der Formel IIb

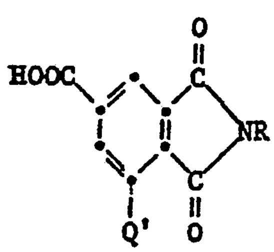

(IIb)

in Gegenwart einer Base mit einer Verbindung der Formel IIIa

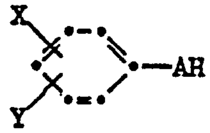

(IIIa)

oder mit einem Salz davon zu einer Verbindung der Formel IVb

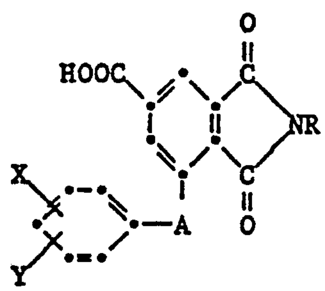

(IVb)

umsetzt, die Verbindung der Formel IVb durch Hydrolyse in die Dicarbonsäure überführt und diese Dicarbonsäure in das Anhydrid überführt, welches zu einer Verbindung der Formel Id

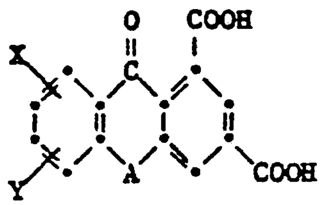

(Id)

cyclisiert wird und diese Verbindung der Formel Id gegebenenfalls anschliessend in eine Verbindung der Formel I überführt, worin E und E'-COOR" oder -CON(R")$_2$ darstellen, wobei A, X, und Y die unter Formel I angegebene Bedeutung haben und Q' -$NO_2$ oder eine Halogenatom bedeuten.

Zur Herstellung von Verbindungen der Formel I, worin E und E' in 1,3- oder 3,4-Stellung gebunden sind, verwendet man vorzugsweise Verbindungen der Formel II bzw. IIb mit Q' = -$NO_2$ Für die Herstellung von Verbindungen der Formel I, worin E und E' in 1,2- oder 2,3- Stellung gebunden sind, setzt man bevorzugt Verbindungen der Formel II mit Q' = Chlor ein.

Die Umsetzung der Verbindungen der Formeln II oder IIa bzw. IIb mit den Verbindungen der Formeln III oder IIIa bzw. deren Salzen wird mit Vorteil in Gegenwart eines inerten organischen Lösungsmittels vorgenommen, wie z. B. N,N-Dialkylamiden von aliphatischen Monocarbonsäuren mit 1 - 3 C-Atomen im Säureteil, z. B. N,N-Dimethylformamid und N,N-Dimethylacetamid; Dialkylsulfoxiden, z. B. Dimethylsulfoxid und Diäthylsulfoxid; aliphatischen oder cyclischen Äthern, z. B. Diäthyläther, Di-isopropyläther, Dimethoxyäthan, Tetrahydrofuran und Dioxan; cyclischen Amiden, wie N-Methylpyrrolidon oder N-Äthylpyrrolidon. Bevorzugte Lösungsmittel sind N,N-Dimethylformamid und Tetrahydrofuran.

Als Salze von Verbindungen der Formel III kommen sowohl Salze mit organischen als auch anorganischen Basen in Betracht. Bevorzugt sind Alkalimetall- und quaternäre Ammoniumsalze, wie die Na-, K-, Tetramethyl-, Tetraäthyl-, Benzyltrimethyl- und Benzyltriäthylammoniumsalze. Die genannten Salze können als solche eingesetzt oder auf an sich bekannte Weise in situ gebildet werden. Bevorzugt setzt man die Verbindungen der Formel III in Form ihrer Dinatriumsalze ein. Werden die Verbindungen der Formel III als freie Säuren eingesetzt, so wird die Umsetzung in Gegenwart einer organischen oder anorganischen Base, wie Triäthylamin, Natrium- oder Kaliumfluorid oder -carbonat vorgenommen.

Die Cyclisierung der Verbindungen der Formeln IV, IVa und IVb wird vorzugsweise in Gegenwart einer Protonensäure oder einer Lewis-Säure vorgenommen. Beispiele geeigneter Protonensäuren sind Polyphosphorsäure, gegebenenfalls im Gemisch mit Phosphoroxychlorid, Chlorsulfonsäure und Schwefelsäure. Geeignete Lewis-Säuren sind z. B. Aluminiumtrichlorid oder Bortrifluorid. Bevorzugt ist die Cyclisierung in Gegenwart von Polyphosphorsäure oder Aluminiumtrichlorid. Wird die Cyclisierung in Gegenwart einer Lewis-Säure, wie Aluminiumtrichlorid, durchgeführt, so arbeitet man zweckmässig in Gegenwart eines inerten organischen Lösungsmittels. Als

solche kommen vor allem chlorierte aliphatische oder aromatische Kohlenwasserstoffe, wie Dichlormethan, 1,2-Dichloräthan, 1,2,3-Trichlorpropan, 1,1,2,2-Tetrachloräthan, Chlorbenzol und Dichlorbenzole; Nitromethan, Nitrobenzol und Schwefelkohlenstoff in Betracht. Wird als Cyclisierungsmittel eine Protonensäure verwendet, so arbeitet man zweckmässig in einem Überschuss an Säure, vor allem überschüssiger Polyphosphorsäure.

Die Überführung der Verbindungen der Formeln Ib in Verbindungen der Formel I mit anderer Bedeutung von R oder in Verbindungen der Formeln I oder Id, worin E und E' unabhängig voneinander -COOR", $-CON(R")_2$, -CN oder zusammen -CO-O-CO- darstellen, kann auf an sich bekannte Weise vorgenommen werden, z. B. wie folgt: E/E' = -COOH oder zusammen -CO-O-CO-: durch Hydrolyse in Gegenwart geeigneter Basen, wie KOH oder NaOH und anschliessende Cyclisierung in Gegenwart üblicher Dehydratisierungsmittel, wie Acetanhydrid, oder durch Erhitzen, gegebenenfalls in einem geeigneten Lösungsmittel, wie Xylolen; E/E' = $-CON(R")_2$: durch Umsetzung mit geeigneten primären oder sekundären Aminen; E/E' = -COOR" mit R"≠ Wasserstoff: durch Umsetzung mit geeigneten Alkoholen; Verbindungen der Formel mit anderer Bedeutung von R: durch Umsetzung der Anhydride mit Aminen $NH_2$-R oder durch Umsetzung von Verbindungen der Formel I/Ib, worin R Wasserstoff ist, mit entsprechenden Halogeniden; E/E' = -CN: durch Umsetzung der entsprechenden Carbonsäuren mit Ammoniak in Gegenwart von Dehydratisierungs-Katalysatoren oder durch Behandlung der entsprechenden Säureamide mit wasserabspaltenden Mitteln, wie $P_2O_5$; Q = $-OCOCH{=}CH_2$ oder $-OCOC(CH_3){=}CH_2$: durch Umsetzung der entsprechenden w-Hydroxyalkylimide mit Acrylsäurechlorid oder Methacrylsäurechlorid.

Verbindungen der Formel Ic können gewünschtenfalls auf an sich bekannte Weise zu den Carbonsäureamiden oder Carbonsäuren hydrolysiert werden.

In Verbindungen der Formeln I, Ib, Ic, Id oder IV können Gruppen X, Y und Z auch nach an sich bekannten Methoden in davon verschiedene Gruppen X, Y oder Z übergeführt werden. So können z. B. Nitrogruppen X, Y oder Z durch Umsetzung mit Mercaptanen oder Salzen davon in Gruppen -SR' umgewandelt werden, oder Nitrogruppen Z können zu Aminogruppen reduziert oder durch Umsetzung mit Alkalimetallcarbonaten oder -acetaten in OH-Gruppen übergeführt werden. Für die allfällige Umwandlung der Verbindungen der Formel IV in die Säurechloride können an sich bekannte Chlorierungsmittel, wie Thionylchlorid, Phosgen oder bevorzugt Oxalylchlorid eingesetzt werden.

Für die Verbindungen der Formeln IV und IVa gilt in bezug auf bevorzugte Bedeutungen von A, X, Y, Z und R das oben Angegebene. Die Ausgangsprodukte der Formeln II IIa und III sind bekannt oder können nach an sich bekannten Methoden hergestellt werden. Von den Verbindungen der Formel IVc

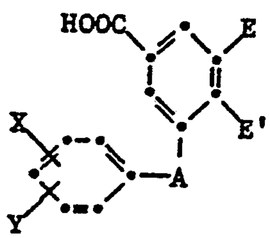

(IVc)

wird diejenigen hervorzuheban indenen X und Y die zuvor angegebene Bedeutung haben und E und E' zusammen für -CO-O-CO- oder -CO-NR-CO- stehen.

Die Verbindungen der Formel I finden z. B. Anwendung als Photosensibilisatoren für lichtvernetzbare Polymere der verschiedensten Art. Sie zeichnen sich durch eine hohe Lichtempfindlichkeit und eine gute Verträglichkeit mit den Polymeren aus. Besonders die Thioxanthonanhydride und -imide der Formel I eignen sich dank der unerwarteten bathochromen Verschiebung ihrer Adsorption für Anwendungen, bei denen mit langwelligem UV-Licht (bis ca. 450 nm) bestrahlt wird.

Unter der Einwirkung von Licht vernetzbare Polymere werden z. B. zur Herstellung von Druckplatten für das Offsetdruckverfahren, zur Herstellung von Photo-Offset-Lacken, für die unkonventionelle Photographie, z. B. zur Herstellung von photographischen Bildern mittels Photopolymerisation oder Photovernetzung, verwendet. Solche Polymere finden insbesondere Anwendung als sogenannte Photoresists zur Herstellung von gedruckten Schaltungen nach an sich bekannten Methoden. Dabei wird die mit der lichtempfindlichen Schicht versehene Seite der Leiterplatte durch ein das Leiterbild aufweisendes Dianegativ belichtet und dann entwickelt, worauf man die unbelichteten Stellen der Schicht durch Entwicklungsflüssigkeit heraushoIt. Solche Anwendungen, einschliesslich der besonders geeigneten, lichtempfindlichen Polymeren, sind z. B. in der DE-A-3 117 568 beschrieben.

Die Verbindungen der Formel I, besonders die Thioxanthonanhydride und -imide, können auch als Sensibilisatoren für photohärtbare, gegebenenfalls gefärbte Massen eingesetzt werden. Derartige Massen enthalten vorzugsweise ein photopolymerisierbares Bindemittel, besonders ein olefinisch ungesättigtes Bindemittel, gegebenenfalls ein Pigment oder einen Farbstoff und einen Photoinitiator, z. B. einen solchen der Formel V

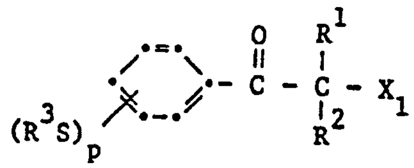

(V)

worin p die Zahl 2 und insbesondere 1 ist, $R^3$ Wasserstoff, $C_1$-$C_{12}$-Alkyl, $C_3$-$C_6$-Alkenyl, $C_5$-$C_6$-Cycloalkyl, $C_7$-$C_9$-Phenylalkyl, Phenyl, durch -COO($C_1$-$C_4$Alkyl) substituiertes Phenyl oder eine der Gruppen $-CH_2CH_2OH$, $-CH_2CH_2-OOC-CH=CH_2$, $-CH_2CN$, $-CH_2COOH$, $-CH_2COO(C_1-C_8\text{-Alkyl})$, $-CH_2CH_2CN$, $-CH_2CH_2COO(C_1-C_8\text{-Alkyl})$,

$-CH_2CH_2CN$, $-CH_2CH_2COO(C_1-C_8\text{-Alkyl})$,

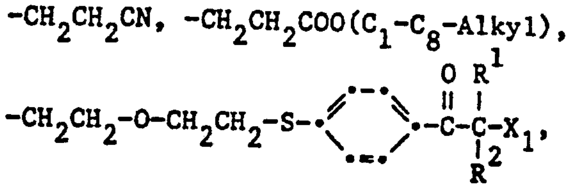, $-CH_2CH_2-O-CH_2CH_2-SH$

oder

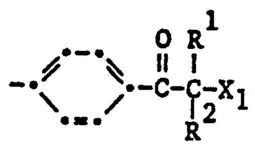

bedeutet,

$R^1$ und $R^2$ unabhängig voneinander $C_1$-$C_4$-Alkyl, Phenyl oder $C_7$-$C_9$-Phenylalkyl sind oder $R^1$ und $R^2$ zusammen $C_2$-$C_8$-Alkylen sind und
$X_1$ eine Aminogruppe $-N(R^4)(R^5)$ ist, worin
$R^4$ $C_1$-$C_8$-Alkyl, durch OH, $C_1$-$C_4$-Alkoxy oder CN substituiertes $C_2$-$C_4$-Alkyl oder $C_3$-$C_5$-Alkenyl ist und
$R^5$ eine der für $R^4$ gegebenen Bedeutungen hat oder zusammen mit $R^4$ $C_4$-$C_5$-Alkylen bedeutet, das durch -O-, -S- oder $-N(R^6)-$ unterbrochen sein kann, wobei $R^6$ $C_1$-$C_4$-Alkyl, 2-Cyanoäthyl, 2-Hydroxyäthyl oder 2-Hydroxypropyl ist.

Bevorzugt sind Verbindungen der Formel V, worin p die Zahl 1 ist $R^3S$- in 4-Stellung gebundenes Methylthio oder 2-Hydroxyäthylthio, $R^1$ und $R^2$ je Methyl oder Äthyl und $X_1$ die Morpholinogruppe bedeuten. Die Verbindungen der Formel V können in Analogie zu den aus EP-A-3 002 bekannten Methoden durch Einführung der Aminogruppe $X_1$ in entsprechende schwefelhaltige Phenylalkylketone hergestellt werden. Der Anteil an Photoinitiator in den photohärtbaren Massen beträgt zweckmässig 0,1 - 20 Gew.-%, bevorzugt 1 - 6 Gew.-%.

Geeignete photopolymerisierbare Bindemittel (photopolymerisierbare Verbindungen) sind beispielsweise in der schon erwähnten DE-A-3 117 568 beschrieben. Die gegebenenfalls mitzuverwendenden Pigmente können anorganisch oder organisch sein, wie Titandioxid, Russ oder Metallpulver, Mono- oder Disazopigmente, Phthalocyaninpigmente oder Pigmente der Perylen-, Thioindigo-, Chinacridon- oder Triphenylmethanreihe. Als Farbstoffe kommen z. B. Azofarbstoffe, Azomethinfarbstoffe, Anthrachinonfarbstoffe oder Metallkomplexfarbstoffe in Betracht.

Gegebenenfalls gefärbte photohärtbare Massen der oben beschriebenen Art können für verschiedene Zwecke eingesetzt werden, vor allem für Druckfarben, insbesondere für den Offsetdruck, den Siebdruck oder den den Tiefdruck, ferner als Anstrichsmittel oder Photoresists.

Ein weiteres wichtiges Anwendungsgebiet der Verbindungen der Formel I ist deren Einsatz als Photoredoxkatalysatoren bei verschiedenen Oxidations-/Reduktionsreaktionen oder in lichtempfindlichen Beschichtungsmaterialien. Besonders eignen sie sich aber zusammen mit Polymeren mit H-Donorgruppen zur Bilderzeugung durch Lichteinwirkung auf verschiedenen organischen oder anorganischen Substraten, vor allem zur Erzeugung von elektrisch leitenden Überzügen oder Mustern, besonders gedruckten Schaltungen, mittels stromloser Metallabscheidung.

Geeignete Substrate für die Bilderzeugung sind z. B. Glas, Metalle und Metalloxide, wie Aluminium, Aluminiumoxid und Kupfer, Keramik, Papier und hochmolekulare organische Materialien. Als hochmolekulare organische Materialien kommen natürliche und synthetische Polymere in Betracht, z. B. Cellulosematerialien, wie Celluloseacetate, Cellulosepropionate, Cellulosebutyrate und Celluloseäther, wie Methylcellulose; von α, β-ungesättigten Säuren abgeleitete Polymere, wie Polyacrylate und Polymethacrylate, Polyacrylamide und Polyacrylnitril; Styrolpolymere und Copolymere davon, z. B. Styrol/Butadiencopolymere und Acrylnitril/Butadien/Styrolcopolymere; Vinyl- und Vinylidenpolymere und Copolymere davon, wie Polyvinylchlorid, Polyvinylidenchlorid, Vinylchlorid/Vinylidenchloridcopolymere, Vinylchlorid/Vinylacetatcopolymere; von ungesättigten Alkoholen und Aminen abgeleitete Polymere und Derivate davon, wie Polyvinylalkohol, Polyvinylacetat und Polyallylmelamin; vernetzte Epoxidharze; Polyacetale; Polyalkylenoxide und Polyphenylenoxide; Polyamide, Polyimide, Polyamid-Polyimid-Blockcopolymere, Polysulfone und Polyester; Alkydharze, z. B. Glycerin/Phthalsäureharze und deren Mischungen mit Melamin/Formaldehydharzen; Melamin-Formaldehyd-, Harnstoff-Formaldehyd-, Phenol-Formaldehydharze.

Als Polymere mit H-Donorgruppen kommen Polymerisations-, Polykondensations- oder Polyadditionsprodukte

mit freien OH-, NH- oder Alkylaminogruppen, besonders $-N(CH_3)_2$-Gruppen, in Betracht, wie Polymerisationsprodukte aus (Meth)acrylsäurehydroxyalkylestern oder -hydroxyalkylamiden mit anderen olefinisch ungesättigten Monomeren, wie (Meth)acrylsäure, (Meth)acrylsäureestern und (Meth)acrylsäurenitril; ferner teilweise verseifte Celluloseacetate oder Gelatine, und Additionsprodukte von Diphenolen, Dicarbonsäuren oder Diaminen und Diglycidyläthern, besonders gegebenenfalls vorverlängerten Diglycidyläthern von Bispehnol A.

Zur Erzeugung von elektrisch leitenden Überzügen oder Mustern mittels stromloser Metallabscheidung werden Gemische aus Verbindungen der Formel I und Polymeren mit H-Donorgruppen und gegebenenfalls Metallsalzen der Gruppen Ib und VIII des Periodischen Systems, besonders Kupfersalzen, belichtet. Dabei beträgt der Anteil an Verbindung der Formel I zweckmässig 0,1 bis 20 Gew.-%, bezogen auf das Gemisch aus Polymer und Verbindung der Formel I. Unter Lichteinwirkung werden die Verbindungen der Formel I zu Radikalen reduziert, die ihrerseits andere Verbindungen, wie die genannten Metallsalze reduzieren können. Auf den dabei erzeugten Radikalen bzw. nullwertigen nicht-leitenden Metallkeimen (nicht-leitenden sichtbaren Bildstellen) können dann in üblicher Weise durch stromlose Abscheidung von Metallen, wie Kupfer, Nickel, Kobalt, Silber, Zinn usw., elektrisch leitende metallische Überzüge oder Muster hergestellt werden. Diese metallischen Überzüge oder Muster können gewünschtenfalls durch elektrolytische Metallabscheidung mit konventionellen Metallabscheidungsbädern verstärkt werden. Bisher war nicht bekannt, dass Xanthone und Thioxanthone photochemisch Metallionen der genannten Art reduzieren können. Verbindungen der Formel I mit geeigneten funktionellen Gruppen, z. B. Anhydridgruppen oder Gruppen $-(-CH_2)_n-OCOCH{=}CH_2$ und $-(CH_2)_n-OCOC(CH_3){=}CH_2$, können auch chemisch in Polymere eingebaut bzw. mit anderen äthylenisch ungesättigten Monomeren polymerisiert werden. Weisen derartige Polymere H-Donorgruppen auf, zweckmässig in etwa 20 % der wiederkehrenden Strukturelemente, so können sie ebenfalls wie oben beschrieben für die Bilderzeugung mittels stromloser Metallabscheidung (mit oder ohne Zusatz von Metallsalzen der Gruppen Ib und VIII des Periodischen Systems) eingesetzt werden.

Für die Belichtung können bei den oben erwähnten Anwendungen an sich beliebige geeignete Lichtquellen eingesetzt werden, z. B. Xenonlampen, Metallhalogenidlampen und besonders Quecksilberhoch- und -mitteldrucklampen.

**Beispiel 1:**

a) 40,0 g 3-Nitrophthalimid und 49,5 g Thiosalicylsäure-Dinatriumsalz [hergestellt durch Lösen von Thiosalicylsäure in 2 Äquivalenten 1 N Natriumhydroxidlösung und Eindampfen, am Schluss zweimal mit Xylol] werden in 200 ml N,N-Dimethylformamid (DMF) während 8 Std. bei 80°C gerührt. Das Gemisch wird am Rotationsverdampfer bei 70°C eingedampft und der Rückstand wird mit 2 N Salzsäure unter Rühren zerrieben. Der Niederschlag wird abfiltriert, mit Wasser gewaschen und getrocknet. Nach dem Umkristallisieren aus Dioxan erhält man 50,2 g (81 % d.Th.) 3-(2-Carboxyphenylthio)-phthalimid; Smp. 289 - 290°C.

Analyse für $C_{15}H_9NO_4S$ (Molgewicht 299,30):

| | | | | | |
|---|---|---|---|---|---|
| berechnet | C 60,19 | H 3,03 | N 4,67 | O 21,38 | S 10,71 % |
| gefunden | C 59,55 | H 3,16 | N 4,57 | O 21,49 | S 10,46 %. |

b) 20,0 g (66,8 mMol) 3-(2-Carboxyphenylthio)-phthalimid werden in 130 g Polyphosphorsäure suspendiert und während 90 Min. bei 150°C gerührt. Das Reaktionsgemisch wird abgekühlt, vorsichtig mit 500 ml Wasser verrührt, und der Niederschlag wird abfiltriert und mehrmals mit Wasser gewaschen. Nach dem Trocknen im Vakuumtrockenschrank bei 150°C wird aus Xylol umkristallisiert. Man erhält 12,4 g (66 % d.Th.) Thioxanthon-3,4-dicarbonsäureimid; Smp. 348 - 350°C.

Analyse für $C_{15}$-$H_7NO_3S$ (Molgewicht 281,29):

| | | | | | |
|---|---|---|---|---|---|
| berechnet | C 64,05 | H 2,51 | N 4,98 | O 17,06 | S 11,40 % |
| gefunden | C 63,87 | H 2,79 | N 4,98 | O 17,17 | S 11,25 %. |

**Beispiel 2:**

a) 15 g (72,7 mMol) 3-Nitrophthalsäure-N-methylimid und 18,1 g (91 mMol) Thiosalicylsäure-Dinatriumsalz werden in 750 ml Tetrahydrofuran (THF) einen Tag am Rückfluss gerührt. Nach dem Abkühlen wird mit 2N Salzsäure und Toluol versetzt. Die organischen Extrakte werden getrocknet und eingedampft. Nach dem Umkristallisieren aus Dioxan erhält man 15,54 g (68 % d.Th.) 3-(2-Carboxyphenylthio)-phthalsäure-N-methylimid; Smp. 270 - 272°C.

Analyse für $C_{16}H_{11}NO_4S$ (Molgewicht 313,33):

| | | | | |
|---|---|---|---|---|
| berechnet | C 61,34 | H 3,54 | N 4,47 | S 10,23 % |
| gefunden | C 61,00 | H 3,60 | N 4,80 | S 9,90 %. |

b) 15,5 g (49,5 mMol) 3-(2-Carboxyphenylthio)-phthalsäure-N-methylimid werden durch Kochen mit 400 ml THF und 150 ml Oxalylchlorid in das Säurechlorid übergeführt. Nach 5 h am Rückfluss wird das Gemisch mit 150 ml Chlorbenzol versetzt und auf ein Totalvolumen von 150 ml eingedampft. Zur abgekühlten Suspension werden 13,2 g (98,9 mMol) wasserfreies Aluminiumchlorid gegeben. Das Gemisch wird 18 h bei 25°C gerührt und dann eingedampft. Der Rückstand wird in 2N Salzsäure aufgenommen und mit THF/Toluol extrahiert. Die Extrakte werden mit gesättigter $NaHCO_3$-Lösung gewaschen, über Natriumsulfat getrocknet und eingedampft. Nach dem Umkristallisieren aus Dioxan erhält man 5 g (34 % d.Th.) Thioxanthon-3,4-dicarbonsäure-N-methylimid; Smp. 287 - 288°C.

Analyse für $C_{16}H_9NO_3S$ (Molgewicht 295,31):

| | | | | |
|---|---|---|---|---|
| berechnet | C 65,08 | H 3,07 | N 4,74 | S 10,86 % |
| gefunden | C 65,03 | H 3,16 | N 4,75 | S 10,65 %. |

**Beispiel 3:**

4,80 g (17,1 mMol) Thioxanthon-3,4-dicarbonsäureimid werden in 511 ml 0,1 N NaOH-Lösung während 90 Minuten am Rückfluss gehalten. Das entstandene Gemisch aus Amidsäure- und Dicarbonsäure-Natriumsalzen wird mit konz. Salzsäure angesäuert und 18 h am Rückfluss gehalten. Die rohe Thioxanthon-3,4-dicarbonsäure wird abfiltriert, mit Wasser gewaschen und in Xylol mit 11 ml Acetanhydrid durch Kochen am Rückfluss ins Anhydrid übergeführt, das nach teilweisem Eindampfen der Lösung ausfällt. Man erhält 4,32 g (90 % d.Th.) Thioxanthon-3,4-dicarbonsäureanhydrid; Smp. 330 - 301°C.

Analyse für $C15\text{-}H_6O_4S$ (Molgewicht 282,27):

| | | | | |
|---|---|---|---|---|
| berechnet | C 63,83 | H 2,14 | O 22,67 | S 11,36 % |
| gefunden | C 63,97 | H 2,01 | O 22,61 | S 11,13 %. |

**Beispiele 4 - 14:**

2 g (7,1 mMol) Thioxanthon-3,4-dicarbonsäureanhydrid, 0,92 g (7,1 mMol) 2-Äthyl-1-hexylamin und 20 ml Xylol werden 30 Min. unter Wasserabscheidung am Rückfluss gehalten. Beim Abkühlen fallen 2,35 g (85 % d.Th.) Thioxanthon-3,4-dicarbonsäure-N-(2-äthyl-n-hexyl)imid aus; Smp. 189 - 190°C.

Analyse für $C_{23}H_{23}NO_3S$ (Molgewicht 393,50):

| | | | | |
|---|---|---|---|---|
| berechnet | C 70,20 | H 5,89 | N 3,56 | S 8,15 % |
| gefunden | C 70,05 | H 5,57 | N 3,54 | S 8,17 %. |

Auf analoge Weise werden die in der folgenden Tabelle I angegebenen Thioxanthon-3,4-dicarbonsäureimide hergestellt. Abweichende Reaktionsbedingungen sind ebenfalls in der Tabelle angegeben.

**Tabelle I:**

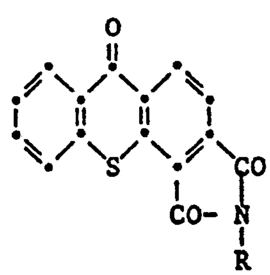

| Bsp. Nr. | R | Ausbeute % d.Th. | Smp. °C | Bemerkungen |
|---|---|---|---|---|
| 5 | $-C(CH_3)_2-CH_2-C(CH_3)_3$ | 77 | 237 - 41 | Reaktion in o-Dichlorbenzol |
| 6 | 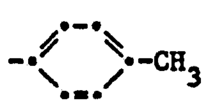 | 49 | 296 - 99 | umkristallisiert aus Dioxan |

| | | | | |
|---|---|---|---|---|
| 7 | $-CH_2CH_2OH$ | 82 | 287 - 89 | do. |
| 8 | $-CH_2CH_2N(CH_3)_2$ | 80 | 235 - 36 | - |
| 9 | $-N(CH_3)_2$ | 83 | 307 - 8 | - |
| 10 | $-CH_2CH_2SO_3^-Na^+$ | 84 | > 330 | umkristallisiert aus $H_2O$/THF |
| 11 | $-CH_2CH_2CH_2N(CH_3)_2$ | 45 | 186 - 8 | - |
| 12 | $-CH_2CH_2N(_2H_5)_2$ | 81 | 174 - 6 | umkristallisiert aus Dioxan |
| 13 | $-CH-CH=CH_2$ | 75 | 242 - 4 | - |
| 14 | $-CH_2\equiv CCH$ | 57 | > 310 | - |
| 15 | $-CH(CH_3)_2$ um | 81 | 280 - 290 | Reaktion in o-Dichlorbenzol, kristallisiert aus $CH_2Cl_2$/Pentan |

**Beispiel 16:**

30,0 g (106 mMol) Thioxanthon-3,4-dicarbonsäureanhydrid und 265,7 ml 2N NaOH-Lösung werden am Rückfluss bis zur klaren Lösung erhitzt. Nach 45 Min. wird auf 25°C abgekühlt, das Produkt wird abfiltriert, mit Äthanol gewaschen, getrocknet und aus Äthanol/Wasser umkristallisiert. Man erhält das Thioxanthon-3,4-dicarbonsäure-dinatriumsalz (Hemihydrat) in quantitativer Ausbeute; Smp. > 350°C.

Analyse für $C_{15}H_6Na_2SO_5 \cdot 1/2\ H_2O$ (Molgewicht 353,25):

| | | | | |
|---|---|---|---|---|
| berechnet | C 51,00 | H 1,99 | Na 13,00 | S 9,07 % |
| gefunden | C 50,87 | H 2,11 | Na 12,95 | S 8,96 %. |

**Beispiel 17:**

4,0 g (14,2 mMol) Thioxanthon-3,4-dicarbonsäureimid, 4,12 g (21,3 mMol) 1-Octylbromid, 5,89 g (42,7 mMol) Kaliumcarbonat und 40 ml DMF werden 1 Tag bei 80°C gerührt. Das Gemisch wird eingedampft, der Rückstand wird in 2N Salzsäure aufgenommen und mit Methylenchlorid extrahiert. Die organischen Phasen werden mit gesättigter $NaHCO_3$- und gesättigter NaCl-Lösung gewaschen, getrocknet und eingedampft. Nach dem Umkristallisieren aus Cyclohexan erhält man 5,28 g (95 % d.Th.) Thioxanthon-3,4-dicarbonsäure-N-n-octylimid; Smp. 188 - 190°C.

Analyse für $C_{23}H_{23}NO_3S$ (Molgewicht 393,50):

| | | | | | |
|---|---|---|---|---|---|
| berechnet | C 70,20 | H 5,89 | N 3,56 | O 12,20 | S 8,15 % |
| gefunden | C 70,05 | H 5,86 | N 3,71 | O 12,77 | S 8,11 %. |

**Beispiel 18:**

a) 1,47 g (5 mMol) 3,5-Dinitrophthalsäure-N-n-butylimid, 1,24 g (6 mMol) Thiosalicylsäure-dinatriumsalz und 15 ml THF werden 5 h am Rückfluss gerührt. Dann werden 15 ml THF, 10 ml 2N Salzsäure und 30 ml Toluol zugegeben, das Gemisch wird geschüttelt und die organische Phase wird abgetrennt, mit gesättigter NaCl-Lösung gewaschen, getrocknet und eingedampft. Nach dem Umkristallisieren aus THF/Toluol werden 1,80 g (90 % d.Th.) 5-Nitro-3-(2-carboxyphenylthio)-phthalsäure-N-n-butylimid erhalten; Smp. 192 - 196°C.

Analyse für $C_{19}H_{16}N_2O_6S$ (Molgewicht 400,41):

| | | | | |
|---|---|---|---|---|
| berechnet | C 57,00 | H 4,03 | N 7,00 | S 8,01 % |
| gefunden | C 56,82 | H 4,10 | N 6,95 | S 7,72 %. |

b) 20,0 g (50 mMol) 5-Nitro-3-(2-carboxyphenylthio)-phthalsäure-N-n-butylimid werden mit 80 g Polyphosphorsäure 3 h bei 180°C gerührt. Das Gemisch wird abgekühlt und vorsichtig mit 300 ml Wasser versetzt. Der Niederschlag wird abfiltriert, mit Wasser gewaschen und im Vakuum bei 150°C getrocknet. Aus Toluol umkristallisiert werden 12,3 g (64 % d.Th.) 1-Nitrothioxanthon-3,4-dicarbonsäure-N-n-butylimid erhalten; Smp. 264 - 266°C.

Analyse für $C_{19}H_{14}N_2O_5S$ (Molgewicht 382,39):

| | | | | | |
|---|---|---|---|---|---|
| berechnet | C 59,67 | H 3,69 | N 7,33 | O 20,92 | S 8,38 % |
| gefunden | C 59,76 | H 3,86 | N 7,36 | O 20,84 | S 8,19 %. |

**Beispiel 19:**

a) 5,0 g (12 mMol) 5-Nitro-3-(2-carboxyphenylthio)-phthalsäure-N-n-butylimid werden mit 1 g Pd/C (5 Gew.-% Pd) in 100 ml DMF 8 h hydriert. Der Katalysator wird abfiltriert und die Mutterlauge wird eingedampft. Der Rückstand wird in Wasser/THF/Toluol aufgenommen und das Gemisch auf pH 6 gestellt. Die organischen Extrakte werden über Natriumsulfat getrocknet und eingedampft. Aus Methylenchlorid umkristallisiert erhält man 2,34 g (53 % d.Th.) rohe Aminosäure. Zur Charakterisierung wird 1 g (2,7 mMol) der rohen Aminosäure mit 5 ml Acetanhydrid während 30 Min. bei 100°C gehalten, worauf man das Gemisch im Vakuum bei 80°C eindampft. Der Rückstand wird in Salzsäure/THF/Toluol aufgenommen, die organischen Phasen werden mit gesättigter NaCl-Lösung gewaschen, über Natriumsulfat getrocknet und eingedampft. Nach dem Umkristallisieren aus Methylenchlorid/n-Pentan erhält man 800 mg (72 % d.Th.) 5-Acetylamino-3-(2-carboxyphenylthio)-phthalsäure-N-n-butylimid; Smp. 231 - 233°C.

Analyse für $C_{21}H_{20}N_2O_5S$ (Molgewicht 412,46):

| | | | | | |
|---|---|---|---|---|---|
| berechnet | C 61,15 | H 4,89 | N 6,79 | O 19,40 | S 7,77 % |
| gefunden | C 61,00 | H 5,10 | N 6,30 | O 19,10 | S 7,40 %. |

b) 500 mg (1,35 mMol) rohes 5-Amino-3-(2-carboxyphenylthio)-phthalsäure-N-n-butylimid und 5 ml Thionylchlorid werden 1 h am Rückfluss gehalten. Dann werden 10 ml Chlorbenzol zugegeben und das Gemisch wird auf 10 ml Totalvolumen eingedampft. Nach dem Abkühlen fügt man 718 mg (5,39 mMol) wasserfreies Aluminiumchlorid hinzu und rührt 2 h bei 25°C. Das Gemisch wird eingedampft, mit THF/Toluol versetzt und mit Wasser/gesättigter $NaHCO_3$-Lösung auf pH 6 gestellt. Die organischen Extrakte werden mit gesättigter NaCl-Lösung gewaschen, getrocknet und eingedampft. Nach dem Umkristallisieren aus THF erhält man 60 mg (13 % d.Th.) 1-Aminothioxanthon-3,4-dicarbonsäure-N-n-butylimid; Smp. 272 - 274°C.

Analyse für $C_{19}H_{16}N_2O_3S$ (Molgewicht 352,41):

| | | | | |
|---|---|---|---|---|
| berechnet | C 64,76 | H 4,58 | N 7,95 | 0 13,62 % |
| gefunden | C 64,29 | H 4,56 | N 8,02 | O 13,96 %. |

**Beispiel 20:**

a) 20,0 g (49,9 mMol) 5-Nitro-3-(2-carboxyphenylthio)-phthalsäure-N-n-butylimid, 9,08 g (54,9 mMol) n-Decanthiol, 27,6 g (199,6 mMol) Kaliumcarbonat und 500 ml DMF werden 2 h bei 25°C gerührt. Das Gemisch wird im Vakuum eingedampft und der Rückstand wird in Methylenchlorid/verdünnter Salzsäure aufgenommen. Die organischen Phasen werden mit gesättigter NaCl-Lösung gewaschen, über Natriumsulfat getrocknet und eingedampft. Nach dem Umkristallisieren aus Cyclohexan erhält man 27,56 g (93 % d.Th.) 5-n-Decylthio-3-(2-carboxyphenylthio)-phthalsäure-N-n-butylimid; Smp. 113 - 115°C.

Analyse für $C_{29}H_{35}NO_4S_2$ (Molgewicht 525,72):

| | | | | | |
|---|---|---|---|---|---|
| berechnet | C 66,26 | H 6,71 | N 2,66 | S 12,15 | O 12,13 % |
| gefunden | C 66,23 | H 7,01 | N 2,70 | S 12,09 | O 11,97 %. |

b) 11,01 g (21,7 mMol) 5-n-Decylthio-3-(2-carboxyphenylthio)-phthalsäure-N-n-butylimid werden durch einstündiges Kochen mit 55 ml Oxalylchlorid in das entsprechende Säurechlorid übergeführt. Dann werden 120 ml Chlorbenzol zugegeben, und das Gemisch wird auf total 120 ml eingedampft. Nach dem Abkühlen werden 8,68 g (65,1 mMol) wasserfreies Aluminiumchlorid zugegeben. Nach 30 Min. Rühren bei 25°C wird das Gemisch eingedampft, in 2N Salzsäure/THF/Toluol aufgenommen, die organischen Phasen werden mit $NaHCO_3$- und gesättigter NaCl-Lösung gewaschen, getrocknet und eingedampft. Nach dem Umkristallisieren aus Cyclohexan erhält man 10,34 g (95 % d.Th.) 1-n-Decylthiothioxanthon-3,4-dicarbonsäure-N-n-butylimid; Smp. 143 - 145°C.

Analyse für $C_{29}H_{35}NO_3S_2$ (Molgewicht 509,72):

| | | | | |
|---|---|---|---|---|
| berechnet | C 68,33 | H 6,92 | N 2,75 | S 12,58 % |
| gefunden | C 68,30 | H 6,88 | N 2,76 | S 12,28 %. |

**Beispiel 21:**

a) 12,81 g (32 mMol) 5-Nitro-3-(2-carboxyphenylthio)-phthalsäure-N-n-butylimid, 3,88 g (35,2 mMol) Thiophenol, 13,27 g (96 mMol) Kaliumcarbonat und 120 ml DMF werden analog Beispiel 19a) umgesetzt und aufgearbeitet. Nach dem

Umkristallisieren aus Toluol/Cyclohexan erhält man 13,52 g (91 % d.Th.) 5-Phenylthio-3-(2-carboxyphenylthio)-phthalsäure-N-n-butylimid; Smp. 154 - 156°C.
Analyse für $C_{25}H_{21}NO_4S_2$ (Molgewicht 463,57):

| | | | | |
|---|---|---|---|---|
| berechnet | C 64,77 | H 4,57 | N 3,02 | S 12,83 % |
| gefunden | C 64,96 | H 4,73 | N 3,20 | S 13,62 %. |

b) 12,51 g (27 mMol) 5-Phenylthio-3-(2-carboxyphenylthio)-phthalsäure-N-n-butylimid werden analog Beispiel 19b) umgesetzt. Nach dem Umkristallisieren aus Toluol erhält man 11,1 g (92 % d.Th.) 1-Phenylthiothioxanthon-3,4-dicarbonsäure-N-n-butylimid; Smp. 265 - 268°C.

Analyse für $C_{25}H_{19}NO_3S_2$ (Molgewicht 445,55):

| | | | | |
|---|---|---|---|---|
| berechnet | C 67,39 | H 4,30 | N 3,14 | S 14,39 % |
| gefunden | C 67,28 | H 4,29 | N 3,16 | S 14,13 %. |

**Beispiel 22:**

3,0 g (10,2 mMol) Thioxanthon-3,4-dicarbonsäure-N-methylimid werden in einem Druckgefäss vorgelegt und bei -10°C werden 30 ml Dimethylamin dazukondensiert. Das Gemisch wird unter Druck 10 Tage bei 25°C gehalten. Das Dimethylamin wird verdampft. Nach dem Umkristallisieren des Rückstandes aus THF erhält man 2,71 g (78 % d.Th.) 3-N,N-Dimethylcarbamoyl-4-N-methylcarbamoyl-thioxanthon; Zersetzungspunkt zum Edukt ab 185°C.

Analyse für $C_{18}H_{16}N_2O_3S$ (Molgewicht 340,40):

| | | | | | |
|---|---|---|---|---|---|
| berechnet | C 63,52 | H 4,74 | N 8,23 | O 14,10 | S 9,42 % |
| gefunden | C 63,14 | H 4,77 | N 8,01 | O 14,45 | S 9,33 %. |

**Beispiel 23:**

1,0 g (3,5 mMol) Thioxanthon-3,4-dicarbonsäureanhydrid wird mit 20 ml absolutem Äthanol 3 h am Rückfluss gehalten. Das Gemisch wird bei maximal 60°C im Vakuum eingedampft. Der Rückstand wird mit verdünnter Salzsäure und Methylenchlorid versetzt. Die organischen Phasen werden getrocknet und eingeengt. Das 100 MHz-$^1$H-NMR-Spektrum zeigt ein Gemisch der beiden Thioxanthon-3,4-dicarbonsäure-monoäthylester im Verhältnis von ca. 2 : 1 (3- : 4-Ester). Durch Umkristallisation aus Methylenchlorid/n-Pentan wird der 3-Ester praktisch rein erhalten; Ausbeute 790 mg (68 % d.Th.); Smp. 315 - 319°C.

Analyse für $C_{17}H_{12}O_5S$ (Molgewicht 328,34):

| | | | | |
|---|---|---|---|---|
| berechnet | C 62,19 | H 3,69 | O 24,37 | S 9,77 % |
| gefunden | C 61,82 | H 3,77 | O 24,48 | S 9,69 %. |

**Beispiel 24:**

2,9 g (10,3 mMol) Thioxanthon-3,4-dicarbonsäureanhydrid, 58 ml n-Butanol, 15 ml Toluol und 0,87 ml konz. Schwefelsäure werden 20 h am Wasserabscheider gekocht. Das Gemisch wird teilweise eingedampft, und der Rückstand wird in Wasser/Methylenchlorid aufgenommen. Die organischen Extrakte werden mit Sole gewaschen, über Natriumsulfat getrocknet und eingedampft. Der Rückstand wird im Hochvakuum bei 150°C getrocknet, wobei 3,92 g (92 % d.Th.) Thioxanthon-3,4-dicarbonsäure-di-n-butylester vom Smp. 49 - 51°C erhalten werden.

Analyse für $C_{23}H_{24}O_5S$ (Molgewicht 412,50):

| | | | | |
|---|---|---|---|---|
| berechnet | C 66,97 | H 5,86 | O 19,39 | S 7,77 % |
| gefunden | C 67,10 | H 5,88 | O 19,14 | S 7,63 %. |

**Beispiel 25:**

1 g (3,5 mMol) Thioxanthon-3,4-dicarbonsäureanhydrid, 5 ml Diäthylenglykolmonomethyläther und 5 Tropfen konz. Schwefelsäure werden wie in Beispiel 23 beschrieben umgesetzt und das Produkt isoliert. Man erhält 863 mg (48 % d.Th.) Thioxanthon-3,4-dicarbonsäure-bis-[2-(2-methoxyäthoxy)äthyl]ester (flüssig, kristallisiert nach einigen Wochen teilweise).

Analyse für $C_{25}H_{28}O_9S$ (Molgewicht 504,55):

| | | |
|---|---|---|
| berechnet | C 59,51 | H 5,59 % |
| gefunden | C 59,30 | H 5,50 %. |

**Beispiel 26:**

2,30 g (10 mMol) 4,5-Dichlorphthalsäure-N-methylimid, 2,69 g (13 mMol) Thiosalicylsäure-Dinatriumsalz und 12 ml DMF werden 18 h bei 80°C gerührt. Das Gemisch wird in verdünnter Salzsäure/THF/Toluol aufgenommen. Die organischen Phasen werden abgetrennt und mit $NaHCO_3$-Lösung extrahiert. Die basischen wässrigen Extrakte werden angesäuert und mit THF/Toluol extrahiert. Die Extrakte werden getrocknet und eingedampft. Der Rückstand wird aus THF/Toluol fraktioniert kristallisiert. So werden 1,1 g (32 % d.Th.) rohes 4-(2-Carboxyphenylthio)-5-chlorphthalsäure-N-methylimid erhalten. Dieses wird in 12 g Polyphosphorsäure während 10 Min. auf 200°C erhitzt. Das Gemisch wird abgekühlt, in Wasser aufgenommen und mit THF/Toluol extrahiert. Die Extrakte werden mit $NaHCO_3$- und gesättigter NaCl-Lösung gewaschen, über Natriumsulfat getrocknet und eingedampft. Nach dem Umkristallisieren aus THF/Toluol erhält man 580 mg (55 % d.Th.) 4-Chlorthioxanthon-1,2-dicarbonsäure-N-methylimid; Smp. 258 - 266°C.

Analyse für $C_{16}H_8ClNO_3S$ (Molgewicht 329,76):

| | | | |
|---|---|---|---|
| berechnet | C 58,28 | H 2,45 | N 4,25 % |
| gefunden | C 58,53 | H 2,74 | N 4,32 %. |

**Beispiel 27:**

1,65 g (8 mMol) 3-Nitrophthalsäure-N-methylimid, 1,60 g (8,7 mMol) Salicylsäure-Dinatriumsalz und 10 ml N,N-Dimethylacetamid werden 30 Min. bei 120°C gerührt. Nach dem Abkühlen wird das Gemisch in verdünnter Salzsäure/THF/Toluol aufgenommen, die organischen Extrakte werden mit $NaHCO_3$-Lösung extrahiert, die wässrigen basischen Extrakte werden angesäuert und mit THF/Toluol extrahiert. Die Extrakte werden über Natriumsulfat getrocknet und eingedampft, wobei überschüssige Salicylsäure absublimiert. Der Rückstand (1,57 g, max. 5,2 mMol) wird in 14 g Polyphosphorsäure während 90 Min. auf 150°C erhitzt. Das Gemisch wird abgekühlt, in Wasser/THF/Toluol aufgenommen, auf pH 10 gestellt und extrahiert. Die organischen Phasen werden mit $NaHCO_3$-Lösung und gesättigter NaCl-Lösung gewaschen, über Natriumsulfat getrocknet und eingedampft. Aus THF umkristallisiert werden 340 mg (23 % d.Th.) Xanthon-3,4-dicarbonsäure-N-methylimid erhalten; Smp. 280 - 283°C.

Analyse für $C_{16}H_9NO_4$ (Molgewicht 279,25):

| | | | |
|---|---|---|---|
| berechnet | C 68,82 | H 3,25 | N 5,02 % |
| gefunden | C 68,62 | H 3,04 | N 5,22 %. |

**Beispiel 28:**

3,0 g (7,8 mMol) 1-Nitrothioxanthon-3,4-dicarbonsäure-N-n-butylimid und 2,2 g (15,9 mMol) Kaliumcarbonat werden in 35 ml DMF während 1 h bei 50°C gerührt. Das Gemisch wird eingedampft und der Rückstand wird in verdünnter HCl-Lösung aufgenommen und mit THF und Toluol extrahiert. Nach dem Trocknen über Natriumsulfat werden die Extrakte eingedampft. Die Umkristallisation aus THF ergibt 2,04 g (74 % d.Th.) 1-Hydroxythioxanthon-3,4-dicarbonsäure-N-n-butylimid; Smp. 191 - 193°C.

Analyse für $C_{19}H_{15}NO_4S$ (Molgewicht 353,39):

| | | | | |
|---|---|---|---|---|
| berechnet | C 64,58 | H 4,28 | N 3,97 | S 9,07 % |
| gefunden | C 64,29 | H 4,39 | N 3,85 | S 8,83 %. |

**Beispiel 29:**

13,01 g (40 mMol) Thioxanthon-3,4-dicarbonsäure-N-(2-hydroxyäthyl)-imid werden zu einer Lösung von 15,75 g (150,7 mMol) frisch destilliertem Methacrylsäurechlorid in 130 ml absolutem Dioxan zugegeben. Das Gemisch wird mit 2 ml Pyridin versetzt und während 4 h am Rückfluss gehalten. Dann wird das Gemisch bei 50°C im Vakuum eingedampft, der Rückstand wird in Methylenchlorid/Wasser aufgenommen und die organischen Phasen werden getrocknet und eingedampft. Aus Methylenchlorid/n-Pentan werden 11,84 g(71 % d.Th.) Thioxanthon-3,4-dicarbonsäure-N-(2-methacryloyloxyäthyl)-imid erhalten; Smp. 201 - 206°C.

Analyse für $C_{21}H_{15}NO_5S$ (Molgewicht 393,41):

| | | | | |
|---|---|---|---|---|
| berechnet | C 64,12 | H 3,85 | N 3,56 | S 8,15 % |
| gefunden | C 63,39 | H 3,65 | N 3,84 | S 8,04 %. |

**Beispiele 30 und 31:**

1 g (3,7 mMol) 4-Chlorphthalsäure-N-benzylimid, 1,46 g Thiosalicylsäure-Dinatriumsalz und 10 ml DMF werden 4 h am Rückfluss erhitzt. Nach dem Eindampfen wird der Rückstand in $NaHCO_3$-Lösung aufgenommen, diese mit THF/Toluol extrahiert, die Extrakte werden verworfen, dann wird die Wasserphase angesäuert und das Zwischenprodukt wird mit THF/ Toluol extrahiert. Durch Eindampfen erhält man das rohe 4-(2-Carboxyphenylthio)-phthalsäure-N-benzylimid. Dieses wird mit 14,8 g Polyphosphorsäure während 30 Min. bei 200°C gerührt. Das Gemisch wird abgekühlt, mit Wasser verrührt, auf pH 8 gestellt und der Niederschlag abfiltriert und getrocknet. Das Rohprodukt wird mit Methylenchlorid über Kieselgel chromatographiert. Dabei werden die beiden entstandenen Produkte getrennt:

**Thioxanthon 2,3-dicarbonsäure-N-benzylimid,**
120 mg (9 % d.Th.), Smp. 289 - 294°C.
Analyse für $C_{22}$-$H_{13}$-$NO_3S$ (Molgewicht 371,41):

| | | | |
|---|---|---|---|
| berechnet | C 71,15 | H 3,53 | N 3,77 % |
| gefunden | C 71,62 | H 3,91 | N 3,60 %. |

**Thioxanthon-1,2-dicarbonsäure-N-benzylimid;**
40 mg (3 % d.Th.); Smp. 210 - 213°C;
gefunden C 70,78 H 4,17, N 3,66 %.
(Zuordnung mittels $^1$H-NMR-Spektrum).

Das 4-Chlorphthalsäure-N-benzylimid wird durch Erhitzen von 4-Chlorphthalsäureanhydrid (13 g; 71,2 mMol) mit 7,63 g (71,2 mMol) Benzylamin in Xylol am Rückfluss unter Wasserabscheidung hergestellt. Nach dem Eindampfen und Umkristallisieren aus Toluol/Cyclohexan erhält man 18,01 g (93 % d.Th.) 4-Chlorphthalsäure-N-benzylimid; Smp. 117 - 119°C.

Analyse für $C_{15}H_{10}ClNO_2$ (Molgewicht 271,70):

| | | | | |
|---|---|---|---|---|
| berechnet | C 66,31 | H 3,71 | N 5,16 | Cl 13,05 % |
| gefunden | C 65,90 | H 3,80 | N 5,40 | Cl 13,00 %. |

**Beispiele 32 und 33:**

5 g (17,71 mMol) Thioxanthon-3,4-dicarbonsäureanhydrid und 50 ml absolutes Äthanol werden 5 Stunden am Rückfluss gehalten. Das Gemisch wird im Vakuum zur Trockene eingedampft und der Rückstand (Gemisch der beiden Halbester) mit 65 ml Thionylchlorid während 30 Minuten am Rückfluss gehalten. Das Gemisch wird wieder eingedampft und der Rückstand mit 60 ml absolutem Äthanol während 4 Stunden am Rückfluss gehalten. Das Gemisch wird eingeengt, der Rückstand wird in THF/Toluol 1 : 1 gelöst, die Lösung wird mit $NaHCO_3$-Lösung zweimal gewaschen, die organischen Phasen über Natriumsulfat getrocknet und eingedampft. Aus $CH_2Cl_2$/Pentan umkristallisiert, werden 3,4 g (54 %) *Thioxanthon-3,4-di-carbonsäurediätyhlester* erhalten, Smp. 100 - 101°C.

Analog wie der Diäthylester wird der *Thioxanthon-3,4-dicarbonsäuredimethylester* hergestellt, Ausbeute 52 %, Smp. 150 - 55°C.

**Beispiel 34:**

5 g (15,24 mMol) Thioxanthon-3,4-dicarbonsäure-dimethylester werden in 50 ml absolutem Allylalkohol gelöst. Am Rückfluss werden 0,4 g (7,4 mMol) $NaOCH_3$ zugegeben und das Gemisch 1 Tag am Rückfluss gehalten. Die Suspension wird in THF/Toluol 1 : 1 aufgenommen und mit $NaHCO_3$-Lösung dreimal gewaschen. Nach dem Trocknen über Natriumsulfat wird die Lösung eingedampft und der Rückstand aus Methylenchlorid/Pentan umkristallisiert. Ausbeute an *Thioxanthon-3,4-dicarbonsäurediallylester* 3,0 g (52 %), Smp. 45 - 8°C.

**Beispiel 35:**

10,0 g (40 mMol) 5-Carboxy-3-nitrophthalsäure-N-methylimid (hergestellt nach: M.A. Ribi, C.C. Wei und E.H. White, Tetr. *28*, 481 (1972), 100 ml DMSO, 4,85 g (44 mMol) Thiophenol und 17,69 g (128 mMol) wasserfreies Kaliumcarbonat werden während 5 Stunden bei 25° gerührt. Das Gemisch wird auf 300 ml $H_2O$ ausgetragen und angesäuert. Der

Niederschlag wird abfiltriert, mit $H_2O$ gewaschen und in Methylenchlorid aufgenommen. Nach dem Trocknen über Natriumsulfat wird eingedampft und aus Methylenchlorid/Pentan umkristallisiert, Ausbeute 8,0 g (64 %), 5-Carboxy-3-phenylthiophthalsäure-N-methylimid, Smp. 287 - 92°C.

0,50 g (1,6 mMol) des obigen Imids wird mit 3,25 ml (6,5 mMol) 2N NaOH-Lösung während 80 Minuten am Rückfluss gehalten. Das Gemisch wird mit konzentrierter HCl-Lösung stark angesäuert und während 18 Stunden am Rückfluss gehalten. Das Gemisch wird auf Wasser ausgetragen und mit THF/Toluol extrahiert. Die Extrakte werden über $Na_2SO_4$ getrocknet und eingedampft. Der Rückstand (rohe Tricarbonsäure) wird mit 8 ml o-Dichlorbenzol und 0,5 ml Acetanhydrid unter azeotroper Entfernung des Wassers und Eisessigs während 5 Stunden erhitzt. Durch Eindampfen und Zugabe von etwas Pentan werden 0,31 g (65 %) 5-Carboxy-3-phenylthiophthalsäureanhydrid erhalten, Smp. 237 - 47°C.

0,13 g (0,43 mMol) des obigen Anhydrids wird in 1,3 g Polyphosphorsäure während 2 Stunden auf 220°C erhitzt. Das Gemisch wird abgekühlt, mit Wasser verdünnt und mit THF/Toluol extrahiert. Die Extrakte werden über $Na_2SO_4$ getrocknet und eingedampft. Aus THF/Toluol/Pentan umkristallisiert, werden 0,10 g (77 %) Thioxanthon-1,3-dicarbonsäure erhalten, Smp. 325 - 30°C.

Analyse für $C_{15}H_8O_5S$ (Molgewicht 300,28)

| | | | | |
|---|---|---|---|---|
| berechnet | C 60.00 | H 2,69 | O 26,64 | S 10,68 % |
| gefunden | C 59,00 | H 3,23 | O 25,52 | S 10,00 % |

**Anwendungsbeispiel I**

**a) Herstellung des Polymeren**

Es wird ein Polymeres mit folgender Struktur und Zusammensetzung hergestellt:

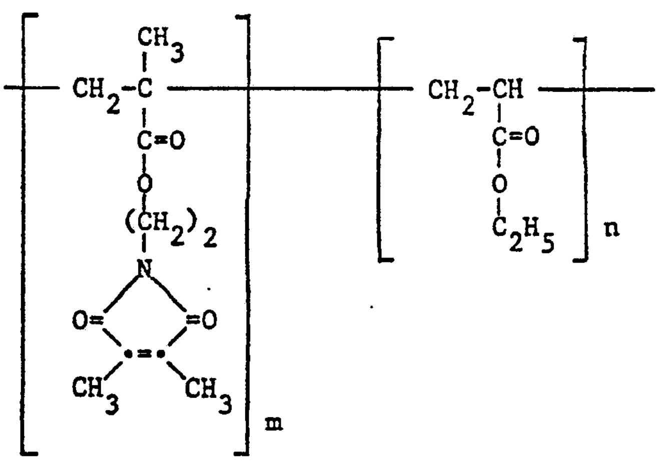

$$\frac{m}{m+n} = 0,8$$

$$\frac{N}{m+n} = 0,2$$

465,5 g (1,963 Mol) Dimethylmaleinimidyl-β-(methacryloyloxy)-äthylester [hergestellt gemäss deutscher Offenlegungsschrift 2.626.769] werden zusammen mit 49,15 g (0,49 Mol) Acrylsäureäthylester unter Stickstoff in 960 ml 1-Acetoxy-2-äthoxyäthan gelöst. Bei 80°C lässt man unter Stickstoffatmosphäre eine Lösung von 3,86 g Azoisobutyronitril in 25 ml 1-Acetoxy-2-äthoxyäthan zulaufen und polymerisiert dann während 6 h. Die noch heisse Lösung wird mit 2,57 g 2,6-Di-tert.-butyl-p-kresol stabilisiert. Das durchschnittliche Molekulargewicht des so erhaltenen Polymeren (bestimmt durch Streulichtmessung in $CHCl_3$) und dessen Grenzviskosität

η grenz betragen:

| durchschnittl. Molekular-Gewicht (Streulicht-messung in $CHCl_3$) | η grenz dl/g | ($CHCl_3$) |
|---|---|---|
| $1,8 \times 10^5$ | 0,17 | 20°C |

**b) Erzeugung von Abbildungen**

Zu 10 g der oben beschriebenen Polymerlösung in 1-Acetoxy-2-äthoxy-äthan, verdünnt mit N,N-Dimethylformamid werden die in der folgenden Tabelle II angegebenen Mengen Sensibilisator zugegeben, wobei sich die Menge (Konzentration) auf den Feststoffgehalt bezieht. Die Polymerlösungen mit dem gelösten Sensibilisator werden durch Aufschleudern (500 Umdrehungen/Min. während 1 Min.) so auf kupferkaschierte Epoxidplatten aufgetragen, dass nach dem Trocknen

(15 Minuten bei 80°C) eine 1 - 3 µ dicke Polymerschicht auf dem Kupfer gebildet wird. Die beschichteten Platten werden durch eine Negativ-Vorlage (Stufenkeil: Stouffer 21-Step-Sensitivity-Guide) mit einer 400 Watt Quecksilberhochdrucklampe im Abstand von 55 cm zum Vakuumtisch belichtet. Vakuumtisch mit vorgeschaltetem Pyrex-Glasfilter von 8 mm Dicke; Belichtungszeiten siehe Tabelle II.

Nach der Belichtung wird das Bild in einem 1,1,1-Trichloräthanbad während 2 Min. entwickelt, wobei die unvernetzten Anteile herausgelöst werden. Das resultierende Reliefbild des abgebildeten Stufenkeils wird durch Ätzen der blanken Kupferteile mit einer 50 %-gen $FeCl_3$-Lösung sichtbar gemacht. In der folgenden Tabelle II bedeutet $S_{rel}$ die relative Empfindlichkeit. Sie ist ein Faktor, der angibt, um wieviel länger oder kürzer als 1 Min. belichtet werden muss, um noch die Stufe 7 (optische Dichte des Stufenkeils = 1) abzubilden. Es gilt folgende Beziehung:

$$S_{rel} = \frac{1}{\sqrt{2}^{\,(7-x)}}$$

wobei X die tatsächlich nach 1 Min. Belichtung abgebildete Stufe bedeutet. Die Bestimmung von $S_{rel}$ basiert auf der von W.S. De Forest ("Photoresist", McGraw Hill Book Company, New York, 1975, Seiten 113 ff.) beschriebenen Methode zur Ermittlung der Photoempfindlichkeit.

**Tabelle II**

| Sensibilisator nach Bsp. Nr. | λ max | ε max | Sensibilisatorkonzentration Gew.-% | Mol.-% | letzte abgebildete Stufe nach 1 Minute | $S_{abs.}$ % | $S_{rel.}$ |
|---|---|---|---|---|---|---|---|
| 1 | 420 | 5200 | 1,41 | 0,005 | 13 | 567 | 8 |
| 2 | 420 | 4800 | 1,48 | 0,005 | 12 | 401 | 5,66 |
| 3 | 423 | 4900 | 1,41 | 0,005 | 8 | 100 | 1,41 |
| 4 | 420 | 4600 | 1,97 | 0,005 | 9 | 200 | 2,00 |
| 5 | 419 | 5000 | 1,97 | 0,005 | 12 | 566 | 5,66 |
| 7 | 420 | 4800 | 1,63 | 0,005 | 12 | 566 | 5,60 |
| 13 | 420 | 4800 | 1,61 | 0,005 | 13 | 800 | 8,00 |
| 14 | 421 | 5200 | 1,6 | 0,005 | 11 | 400 | 4,00 |
| 17 | 420 | 4800 | 1,97 | 0,005 | 12 | 401 | 5,66 |
| 22 | 388 | 6000 | 1,70 | 0,005 | 10 | 201 | 2,83 |
| 24 | 397 | 6080 | 2,06 | 0,005 | 12 | 566 | 5,66 |
| 25 | 398 | 4300 | 2,52 | 0,005 | 12 | 566 | 5,66 |
| 26 | 399 | 3400 | 1,65 | 0,005 | 12 | 566 | 5,66 |

**Anwendungsbeispiel II:** Erzeugung metallischer Bilder.

**a) Herstellung des Matrixpolymeren**

125,9 g Methacrylsäurehydroxypropylester (Gemisch aus 2- und 3-Hydroxypropylester) und 58,3 g Methacrylsäuremethylester werden in 416 ml Dioxan gelöst und unter Rühren und Inertgas auf 70°C erhitzt und dann mit 0,92 g Azoisobutyronitril versetzt. Nach 12 h bei 70°C wird mit 500 ml Dioxan verdünnt. Dann wird das Polymer durch Fällen in 6 Liter Eiswasser isoliert. Ausbeute 169,3 g (91,5 % d.Th.). Tg: 90°C; [η] = 0,65 dl/g in N,N-Dimethylformamid bei 25°C; durchschnittliches Molekulargewicht 120 000 (bestimmt durch Lichtstreuung).

**b) Erzeugung metallischer Bilder**

Das gemäss a) hergestellte Polymer wird in N,N-Dimethylformamid gelöst und mit jeweils 5 Gew.-% der in der folgenden Tabelle III angegebenen Thioxanthonverbindung sowie der äquimolaren Menge Kupfer(II)acetat versetzt. Der Feststoffgehalt der Lösung beträgt 30 Gew.-%. Die Lösung wird dann mit einem Rollercoater als 50 µm starker Nassfilm auf eine Polyesterfolie aufgestrichen und durch eine Maske (21-Step Sensitivity Guide der Fa. Stouffer) auf einem Vakuum-Heiztisch bei 90°C belichtet. Als Lichtquelle dient eine 5 kW Quecksilberhochdrucklampe (Fa. Staub AG, Neu-Isenburg, BRD). Das so erhaltene Bild aus Kupferkeimen wird in einem stromlosen Metallabscheidungsbad der Zusammensetzung:

Kupfersulfat 12 g/l, Formaldehyd 8 g/l, NaOH 15 g/l, Natriumkaliumtartrat 14 g/l, Äthylendiamintetraessigsäure 20 g/l, Octylphenolpolyäthylenglykoläther 1 g/l (n ~ 1; Tryton X 100® der Fa. Röhm & Haas) bei 49°C zu einem metallischen, elektrisch leitenden Bild verstärkt. Die Resultate sind in der Tabelle III angegeben.

**Tabelle III**

| Verbindung nach Beispiel Nr. | Belichtungszeit Sek. | letzte abgebildete Stufe |
|---|---|---|
| 4 | 360 | 8 |
| 17 | 360 | 4 |
| 24 | 360 | 7 |
| 25 | 360 | 2 |

**Anwendungsbeispiel III:** photohärtbare Masse.

Es wird ein Weisslack nach folgender Rezeptur hergestellt:

| | |
|---|---|
| 17,6 g | Ebecryl 593 (Polyesteracrylatharz der Firma UCB, Belgien), |
| 11,8 g | N-Vinylpyrrolidon |
| 19,6 g | Titandioxid RTC-2 (Titandioxid der Firma Thioxide, England), |
| 19,6 g | Sachtolith HDA (Lithopone der Firma Sachtleben Chemie, Duisburg, BRD), |
| 11,8 g | Trimethylolpropan-trisacrylat, |
| 19,6 g | Setalux UV 2276 (acryliertes Epoxidharz auf der Basis von Bisphenol A; Kunstharzfabrik Synthese, Bergen, NL). |

Die obigen Komponenten werden zusammen mit 125 g Glasperlen (Durchmesser 4 cm) in einer 250 ml Glasflasche während mindestens 24 Stunden auf eine Korngrösse ≤ µm gemahlen. In einem Gewichtsteil der so erhaltenen Weisslack-Stammpaste werden 2 Gew.-% des unten angegebenen Photohärters und jeweils 0,25 Gew.-% der unten angeführten Sensibilisatoren (Co-Initiatoren) gelöst. Anschliessend wird das Gemisch nochmals während 16 Std. mit Glasperlen gemahlen.

Die so hergestellten Weisslacke werden mit einem 30 µm Rakel auf Glasplatten aufgetragen. Die Proben werden mit einem PPG-Bestrahlungsgerät mit einer Lampe zu 80 W/cm belichtet, und es wird die unter Erhaltung der Wischfestigkeit erzielbare Härtungsgeschwindigkeit ermittelt.

| Photohärter 2 Gew.-% | Sensibilisator 0,25 Gew.-% | Härtungsgeschwindigkeit |
|---|---|---|
| 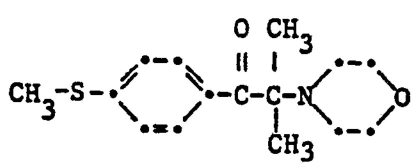 | 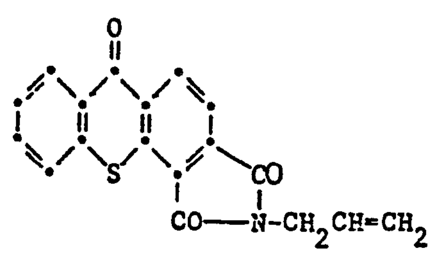 | 90 m / Min. |
| 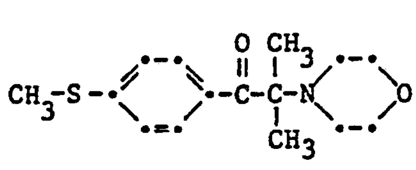 | 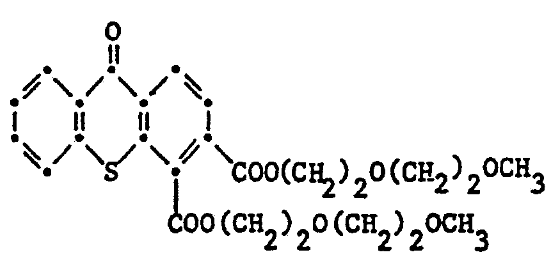 | 60 m / Min. |

Das als Photohärter verwendete 2-Methyl-1-[4-methylthio(phenyl)]-2-morpholino-propanon-1 kann wie folgt hergestellt werden:

151,4 g (0,675 Mol) 3,3-Dimethyl-2-methoxy-2-[4-(methylthio)phenyl]oxiran (Smp. 62 - 64°C) werden in 235,2 g (2,70 Mol) Morpholin gelöst und auf Rückfluss erwärmt.

Nach 15 Stunden wird abgekühlt und das Morpholin abdestilliert. Der Rückstand (Smp. 67 - 71°C) wird in Diäthyläther aufgenommen und mit verdünnter Salzsäure extrahiert. Die Salzsäurelösung wird alkalisch gestellt und mit Diäthyläther extrahiert. Die Ätherlösung wird mit $Na_2SO_4$ getrocknet und eingeengt. Der Rückstand kann aus Äthanol umkristallisiert werden. Smp. 75 - 76°C.

**Patentansprüche**

1. Verbindungen der Formel I

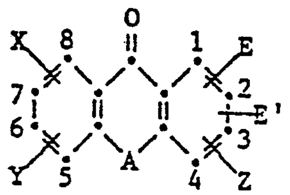

(I)

worin
A -O- oder -S-,
X und Y unabhängig voneinander Wasserstoff, $C_{1-20}$-Alkyl, Halogen, -OR', -SR' oder $-NO_2$'
Z Wasserstoff, $C_{1-4}$-Alkyl, Halogen, -OR', -SR', $-NO_2$, $-NH_2$, -OH oder $-NHCOCH_3$-,
E und E' unabhängig voneinander -COOR", -CON(R")$_2$ oder -CN oder zusammen -CO-O-CO- oder -CO-N(R)-CO-,
R Wasserstoff, $C_{1-20}$-Alkyl, Phenyl, Alkylphenyl mit 1 - 4 C-Atomen im Alkyl, Phenyläthyl, Benzyl, Cyclohexyl, $-(CH_2)_n$-Q, $C_{2-6}$-Alkenyl, Propargyl, $-N(CH_3)_2$, -OH oder $C_{1-10}$-Alkoxy,
R' $C_{1-20}$-Alkyl, Phenyl, Halogenphenyl, Nitrophenyl, Alkyl- oder Alkoxyphenyl mit je 1 - 4 C-Atomen im Alkyl oder Alkoxy, Benzyl oder Phenyläthyl,
die R" unabhängig voneinander Wasserstoff, $M^+$, $C_{1-20}$-Alkyl, Alkoxyalkoxyalkyl mit 3 - 10 C-Atomen oder Hydroxyalkyl mit 2 - 8 C-Atomen,
n die Zahl 1, 2 oder 3,
Q -OH, $-N(C_{1-2}\text{-Alkyl})_2$, $-SO_3^-M^+$, $-OCOCH=CH_2$ oder $-OCOC(CH_3)=CH_2$ und
$M^+$ ein Alkalimetallkation darstellen,
wobei, wenn A -O- darstellt, E und E' zusammen -CO-O-CO- oder -CO-N(R)-CO- bedeuten.

2. Verbindungen nach Anspruch 1 der Formel I, worin E und E' in Nachbarstellung zueinander oder in 1,3-Stellung gebunden sind.

3. Verbindungen nach Anspruch 1 der Formel I, worin X und Y Wasserstoff, A -S- darstellen und Halogenatome Z oder Halogensubstituenten in Z Fluor, Chlor oder Brom, besonders Chlor oder Brom, sind.

4. Verbindungen nach Anspruch 1 der Formel I, worin X und Y Wasserstoff, A -S- und Z Wasserstoff, Chlor, $-NO_2$, $-NH_2$, -OH, -OR' oder -SR' darstellen, E und E' in Nachbarstellung zueinander gebunden sind und unabhängig voneinander eine Gruppe -COOR" oder zusammen -CO-O-CO- oder -CO-N(R)-CO- bedeuten, R die im Anspruch 1 angegebene Bedeutung hat, R'$C_{1-10}$-Alkyl, Benzyl, Phenyl oder Tolyl, die R" unabhängig voneinander Wasserstoff, $M^+$, $C_{1-10}$-Alkyl oder Alkoxyalkoxyalkyl mit 5 - 10 C-Atomen bedeuten.

5. Verbindungen nach Anspruch 1 der Formel Ia

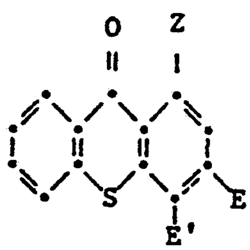

(Ia)

worin Z Wasserstoff, Chlor, $-NO_2$, $-NH_2$, -OH oder -SR', R' $C_{1-10}$-Alkyl oder Phenyl, E und E' unabhängig voneinander -COOR" oder zusammen -CO-O-CO- oder -CO-N(R)-CO-, die R" unabhängig voneinander Wasserstoff, $M^+$, $C_{1-10}$-Alkyl oder Alkoxyalkoxyalkyl mit 5-10 C-Atomen bedeuten und R die im Anspruch 1 angegebene Bedeutung hat.

6. Verbindungen nach Anspruch 5 der Formel Ia, worin Z Wasserstoff, E und E' unabhängig voneinander -COOR" oder zusammen -CO-O-CO- oder -CO-N(R)-CO, R Wasserstoff, $C_{1-10}$-Alkyl, Phenyl, Tolyl, Benzyl, $-(CH_2)_n-OH$, $-(CH_2)_nN(C_{1-2}\text{-Alkyl})_2$ mit n = 2 oder 3, $-CH_2CH_2OCOC(CH_3)=CH_2$, Allyl oder Propargyl und die R" unabhängig voneinander Wasserstoff, $Na^+$, $C_{1-10}$-Alkyl oder $C_{1-2}$-Alkoxy-$C_{1-3}$-Alkoxy-$C_{1-2}$-Alkyl bedeuten.

7. Verbindung nach Anspruch 5 der Formel Ia, worin Z Wasserstoff ist und E und E' zusammen $-CO-N(CH_2CH=CH_2)-CO-$ oder E und E' einzeln COOR mit R gleich Alkyl mit 1 bis 4 C-Atomen oder $-O(CH_2)_2-O-(CH_2)_2-OCH_3$ bedeuten.

8. Verfahren zur Herstellung von Verbindungen der Formel I nach Anspruch 1, worin E und E' in Nachbarstellung zueinander gebunden sind, dadurch gekennzeichnet, dass man eine Verbindung der Formel II

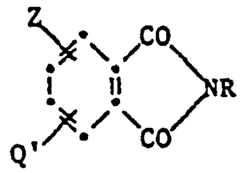

(II)

in Gegenwart einer Base mit einer Verbindung der Formel III

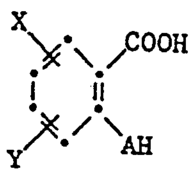

(III)

oder mit einem Salz davon zu einer Verbindung der Formel IV

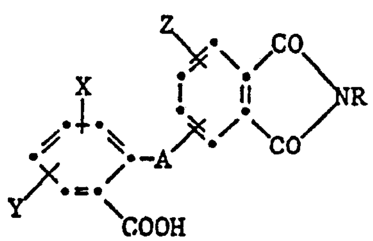

(IV)

umsetzt, die Verbindung der Formel IV, gegebenenfalls unter vorheriger Überführung in das Säurechlorid, zu einer Verbindung der Formel Ib

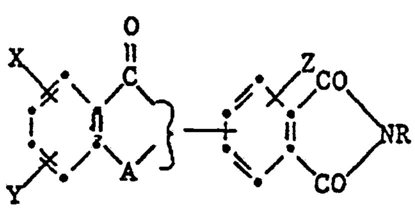

(Ib)

cyclisiert und diese gegebenenfalls anschliessend in eine Verbindung der Formel (I) überführt, worin E und E' unabhängig voneinander -COOR", $-CON(R")_2$, -CN oder zusammen -CO-O-CO- oder -CO-N(R)-CO- mit unterschiedlicher Bedeutung von R darstellen.

9. Verfahren zur Herstellung von Verbindungen der Formel I nach Anspruch 1, worin E und E' unabhängig voneinander -COOR", $-CON(R")_2$ oder -CN darstellen, dadurch gekennzeichnet, dass man eine Verbindung der Formel IIa

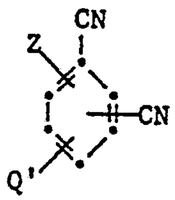

(IIa)

in Gegenwart einer Base mit einer Verbindung der Formel III oder mit einem Salz einer Verbindung der Formel III zu einer Verbindung der Formel IVa

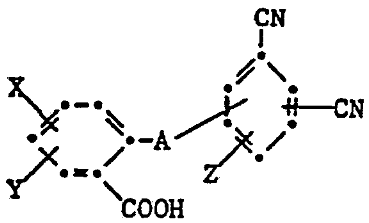

(IVa)

umsetzt, die Verbindung der Formel IVa zu einer Verbindung der Formel Ic

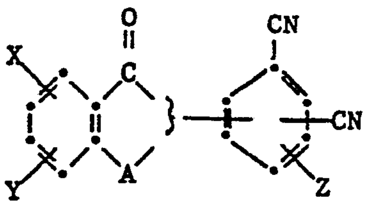

(Ic)

cyclisiert und diese gegebenenfalls anschliessend in eine Verbindung der Formel I überführt, worin E und E' unabhängig voneinander -COOR" oder -CON(R")2 darstellen.

10. Verfahren zur Herstellung von Verbindungen der Formel I nach Anspruch 1, worin E und E' in 1,3-Stellung gebunden sind und COOR" oder CON(R")2 bedeuten und Z = H, dadurch gekennzeichnet, dass man eine Verbindung der Formel IIb

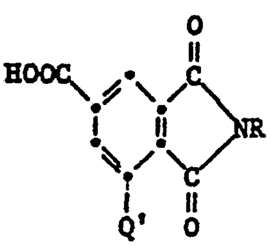

(IIb)

in Gegenwart einer Base mit einer Verbindung der Formel IIIa

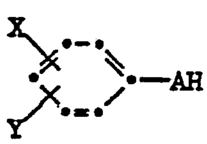

(IIIa)

oder mit einem Salz davon zu einer Verbindung der Formel IVb

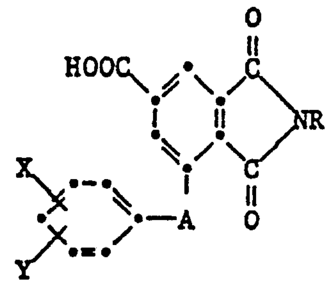

(Ib)

umsetzt, die Verbindung der Formel IVb durch Hydrolyse in die Dicarbonsäure überführt und diese Dicarbonsäure in das Anhydrid überführt, welches zu einer Verbindung der Formel Id

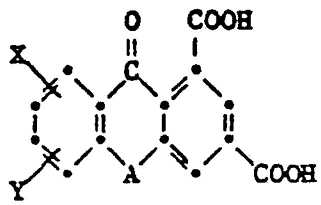

(Id)

cyclisiert wird und diese Verbindung der Formel Id gegebenenfalls anschliessend in eine Verbindung der Formel I überführt, worin E und E'-COOR" oder $-CON(R")_2$ darstellen, wobei A, X und Y die im Anspruch 1 angegebene Bedeutung haben und Q' $-NO_2$ oder ein Halogenatom bedeuten.

11. Verwendung von Verbindungen der Formel I nach Anspruch 1 als Sensibilisatoren für lichtvernetzbare Polymere.

12. Verwendung von Verbindungen der Formel I nach Anspruch 1 als Sensibilisatoren für photohärtbare, gegebenenfalls gefärbte Massen.

**Claims**

1. A compound of the formula I

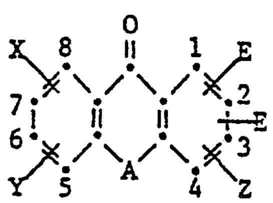

(I)

in which A is -O- or -S-, X and Y independently of one another are hydrogen, $C_{1-20}$-alkyl, halogen, -OR', -SR' or $-NO_2$, Z is hydrogen, $C_{1-4}$-alkyl, halogen, -OR', -SR', $-NO_2$, $-NH_2$, -OH or $-NHCOCH_3$, E and E' independently of one another are -COOR", $-CON(R")_2$ or -CN or together are -CO-O-CO- or -CO-N(R)-CO-, R is hydrogen, $C_{1-20}$-alkyl, phenyl, alkylphenyl having 1 - 4 C atoms in the alkyl moiety, phenethyl, benzyl, cyclohexyl, $-(CH_2)_n-Q$, $C_{2-6}$-alkenyl, propargyl, $-N(CH_3)_2$, -OH or $C_{1-10}$-alkoxy, R' is $C_{1-20}$-alkyl, phenyl, halogeno-phenyl, nitrophenyl, alkyl- or alkoxy-phenyl having in each case 1 - 4 C atoms in the alkyl or alkoxy moiety, benzyl or phenethyl, the radicals R" independently of one another are hydrogen, $M^+$, $C_{1-20}$-alkyl, alkoxyalkoxyalkyl having 3 - 10 C atoms or hydroxyalkyl having 2 - 8 C atoms, n is the number 1, 2 or 3, Q is -OH, $-N(C_{1-2}\text{-alkyl})_2$, $-SO_3\ M^+$, $-OCOCH{=}CH_2$ or $-OCOC(CH_3){=}CH_2$ and $M^+$ is an alkali metal cation, and, if A is -O-, E and E' together are -CO-O-CO- or -CON(R)-CO-.

2. A compound according to claim 1, of the formula I in which E and E' are bonded in the adjacent position to one another or in the 1,3-position.

3. A compound according to claim 1, of the formula I in which X and Y are hydrogen, A is -S- and halogen atoms Z or halogen substituents in Z are fluorine, chlorine or bromine, in particular chlorine or bromine.

4. A compound according to claim 1, of the formula I in which X and Y are hydrogen, A is -S-, Z is hydrogen, chlorine, $-NO_2$, $-NH_2$, -OH, -OR' or -SR', E and E' are bonded in adjacent positions to one another and independently of one another are a -COOR" group or together are -CO-O-CO- or -CO-N(R)-CO, R is as defined in claim 1, R' is $C_{1-10}$-alkyl, benzyl, phenyl or tolyl, and the radicals R" independently of one another are hydrogen, $M^+$, $C_{1-10}$alkyl or alkoxyalkoxyalkyl having 5 - 10 C atoms.

5. A compound according to claim 1, of the formula Ia

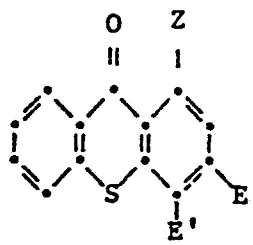

(Ia)

in which Z is hydrogen, chlorine, $-NO_2$, $-NH_2$, -OH or -SR', R' is $C_{1-10}$alkyl or phenyl, E and E' independently of one another are -COOR" or together are -CO-O-CO- or -CO-N(R)-CO-, the radicals R" independently of one another are hydrogen, $M^+$, $C_{1-10}$-alkyl or alkoxyalkoxyalkyl having 5 - 10 C atoms and R is asdefined in claim 1.

6. A compound according to claim 5, of the formula Ia in which Z is hydrogen, E and E' independently of one another are -COOR" or together are -CO-O-CO- or -CO-N(R)-CO-, R is hydrogen, $C_{1-10}$-alkyl, phenyl, tolyl, benzyl, $-(CH_2)n-OH$, $-(CH_2)n-N(C_{1-2}\text{-alkyl})_2$ where n = 2 or 3, $-CH_2CH_2OCOC(CH_3)=CH_2$, allyl or propargyl and the radicals R" independently of one another are hydrogen, $Na^+$, $C_{1-10}$-alkyl or $C_{1-2}$-alkoxy-$C_{1-3}$-alkoxy-$C_{1-2}$-alkyl.

7. A compound according to claim 5, of the formula Ia in which Z is hydrogen and E and E' together are $-CO-N(CH_2CH=CH_2)-CO-$, or E and E' individually are COOR, in which R is alkyl having 1 to 4 C atoms or $-O(CH_2)_2-O-(CH_2)_2OCH_3$.

8. A process for the preparation of a compound of the formula I according to claim 1, in which E and E' are bonded in adjacent positions to one another, which comprises reacting compound of the formula II

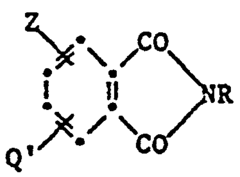

(II)

with a compound of the formula III

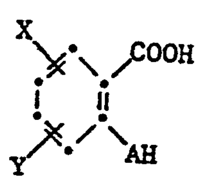

(III)

or with a salt thereof, in the presence of a base to give a compound of the formula IV

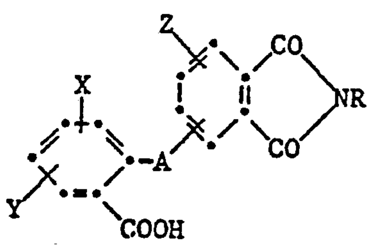

(Ib)

cyclising the compound of the formula IV, if necessary after first converting it into the acid chloride, to give a compound of the formula Ib

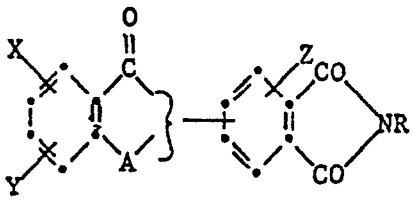

(Ib)

and, if appropriate, then converting this into a compound of the formula (I) in which E and E' independently of one another are -COOR", -CON(R")2 or -CN or together are -CO-O-CO- or -CO-N(R)-CO-, where R has different meanings.

9. A process for the preparation of a compound of the formula I according to claim 1, in which E and E' independently of one another are -COOR", -CON(R")2 or -CN, which comprises reacting a compound of the formula IIa

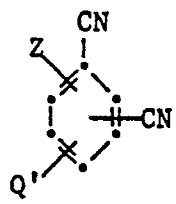

(IIa)

with a compound of the formula III or with a salt of a compound of the formula III, in the presence of a base to give a compound of the formula IVa

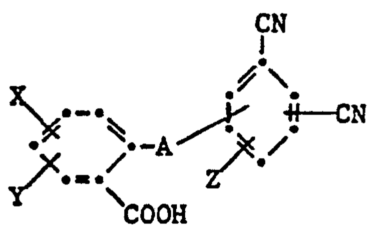

(IVa)

cyclising the compound of the formula IVa to give a compound of the formula Ic

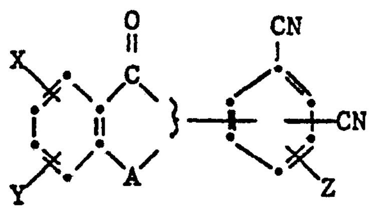

(Ic)

and, if appropriate, then converting this into a compound of the formula I in which E and E' independently of one another are -COOR" or -CON(R")2.

10. A process for the preparation of a compound of the formula I according to claim 1, in which E and E' are bonded in the 1,3-position and are COOR" or -CON(R")2 and Z is H, which comprises reacting a compound of the formula IIb

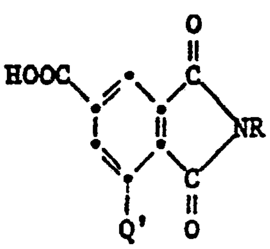

(IIb)

with a compound of the formula IIIa

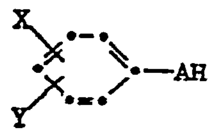

(IIIa)

or with a salt thereof, in the presence of a base, to give a compound of the formula IVb

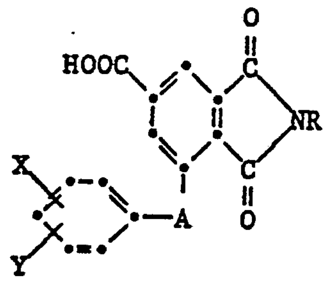

(IVb)

converting the compound of the formula IVb into the dicarboxylic acid by hydrolysis and converting this dicarboxylic acid into the anhydride, which is cyclised to a compound of the formula Id

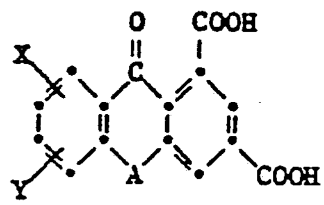

(Id)

and, if appropriate, then converting this compound of the formula Id to a compound of the formula I in which E and E' are -COOR" or -CON(R")2, A, X and Y, Z and R being as defined in claim 1 and Q' being $-NO_2$ or a halogen atom.

11. Use of a compound of the formula I according to claim 1 as a sensitiser for photocrosslinkable polymers.

12. Use of a compound of the formula I according to claim 1 as a sensitiser for photocurable compositions, which may or may not be coloured.

**Revendications**

1. Composés qui répondent à la formule I:

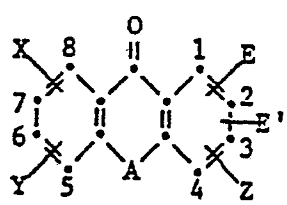

(I)

dans laquelle

A réprésente -O- ou -S-,

X et Y réprésentent chacun, indépendamment l'un de l'autre, l'hydrogène, un alkyle en $C_1$-$C_{20}$, un halogène ou un radical -OR', -SR' ou $-NO_2$,

Z représente l'hydrogène, un alkyle en $C_1$-$C_4$, un halogène ou un radical -OR', -SR', $-NO_2$, $-NH_2$, -OH ou $-NHCOCH_3$,

E et E' représentent chacun, indépendamment l'un de l'autre, un radical -COOR", $-CON(R')_2$ ou -CN, ou forment ensemble un radical -CO-O-CO- ou -CO-N(R)-CO-,

R représente l'hydrogène, un alkyle en $C_1$-$C_{20}$, un phényle, un alkylphényle dont l'alkyle contient de 1 à 4 atomes de carbone, un phényléthyle, un benzyle, un cyclohexyle, un radical $-(CH_2)_n$-Q, un alcényle en $C_2$-$C_6$, un propyne-2 yle (radical "propargyle"), un radical $-N(CH_3)_2$, un radical -OH ou un alcoxy en $C_1$-$C_{10}$,

R' représente un alkyle en $C_1$ à $C_{20}$, un phényle, un halogénophényle, un nitrophényle, un alkyl-phényle ou un alcoxy-phényle qui contiennent chacun de 1 à 4 atomes de carbone dans la partie alkyle ou alcoxy, un benzyle ou un phényléthyle,

les R" représentent chacun, indépendamment l'un de l'autre, l'hydrogène, $M^+$, un alkyle en $C_1$ à $C_{20}$, un alcoxy-alcoxy-alkyle contenant de 3 à 10 atomes de carbone ou un hydroxy-alkyle contenant de 2 à 8 atomes de carbone,

n désigne un nombre égal à 1, à 2 ou à 3,

Q représente un radical -OH, -N(alkyl en $C_1$-$C_2$)$_2$, $-SO_3^-M^+$, $-OCOCH=CH_2$ ou $-OCOC(CH_3)=CH_2$ et $M^+$ représente un cation de métal alcalin,

E et E' pouvant aussi, dans le cas où A désigne -O-, représenter ensemble un radical -CO-O-CO- ou -CO-N(R)-CO-.

2. Composés selon la revendication 1 qui répondent à la formule I dans laquelle E et E' sont en des positions voisines l'une de l'autre ou sont en position 1,3.

3. Composés selon la revendication 1 qui répondent à la formule I dans laquelle X et Y représentent chacun l'hydrogène, A représente -S-, et les atomes d'halogène que peut représenter Z ou oui peuvent se trouver comme substituants dans Z sont le fluor, le chlore et le brome, en particulier le chlore et le brome.

4. Composés selon la revendication 1 qui répondent à la formule I dans laquelle X et Y représentent chacun l'hydrogène, A représente -S-, Z représente l'hydrogène, le chlore ou un radical $-NO_2$, $-NH_2$, -OH, -OR' ou -SR', E et E' sont en des positions voisines l'une de l'autre et représentent chacun, indépendamment l'un de l'autre, un radical -COOR" ou représentent ensemble un radical -CO-O-CO- ou -CO-N(R)-CO-, R a la signification qui lui a été donnée à la revendication 1, R' représente un alkyle en $C_1$-$C_{10}$, un benzyle, un phényle ou un tolyle, et les R" représentent chacun, indépendamment l'un de l'autre, un atome d'hydrogène, $M^+$, un alkyle en $C_1$-$C_{10}$ ou un alcoxy-alcoxy-alkyle contenant de 5 à 10 atomes de carbone.

5. Composés selon la revendication 1 qui répondent à la formule Ia:

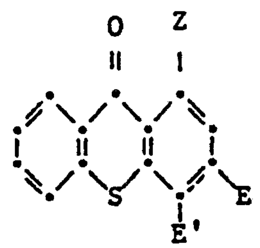

(Ia)

dans laquelle Z représente l'hydrogène, le chlore ou un radical $-NO_2$, $-NH_2$, -OH ou -SR', le symbole R' représente un alkyle en $C_1$-$C_{10}$ ou un phényle, E et E' représentent chacun, indépendamment l'un de l'autre, un radical -COOR" ou forment ensemble un radical -CO-O-CO- ou -CO-N(R)-CO-, les R" représentent chacun, indépendamment l'un de l'autre, un atome d'hydrogène, $M^+$, un alkyle en $C_1$-$C_{10}$ ou un alcoxy-alcoxy-alkyle contenant de 5 à 10 atomes de carbone, et R a la signification qui lui a été donnée à la revendication 1.

6. Composés selon la revendication 5 qui répondent à la formule Ia dans laquelle Z représente l'hydrogène, E et E' représentent chacun, indépendamment l'un de l'autre, un radical -COOR" ou forment ensemble un radical -CO-O-CO- ou -CO-N(R)-CO-, R représente l'hydrogène, un alkyle en $C_1$-$C_{10}$, un phényle, un tolyle, un benzyle, un radical $-(CH_2)_n$-OH ou $-(CH_2)_nN$(alkyl en $C_1$ ou $C_2$)$_2$ dans lequel n est égal à 2 ou 3, ou un radical $-CH_2CH_2OCOC(CH_3)=CH_2$, allyle ou propargyle, et les R" représentent chacun, indépendamment l'un de l'autre, l'hydrogène, $Na^+$, un alkyle en $C_1$-$C_{10}$ ou un radical alcoxy(en $C_1$ ou $C_2$)-alcoxy(en $C_1$ à $C_3$)-alkyle(en $C_1$ ou $C_2$).

7. Composés selon la revendication 5 qui répondent à la formule Ia dans laquelle Z représente l'hydrogène, et E et E' représentent ensemble un radical $-CO-N(CH_2CH=CH_2)-CO-$ ou représentent chacun un radical -COOR dont le symbole R désigne un alkyle contenant de 1 à 4 atomes de carbone ou un radical $-O(CH_2)_2-O-(CH_2)_2-OCH_3$.

8. Procédé pour préparer des composés de formule I selon la revendication 1 dans lesquels E et E' occupent des positions voisines l'une de l'autre, procédé caractérisé en ce qu'on fait réagir un composé de formule II

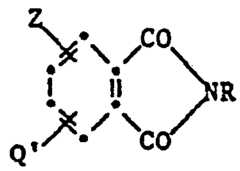

(II)

en présence d'une base, avec un composé de formule III:

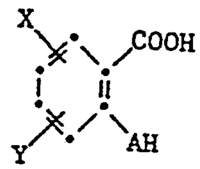

(III)

ou avec un sel d'un tel composé, réaction qui donne un composé de formule IV:

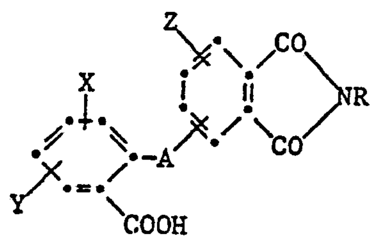

(IV)

on cyclise le composé de formule IV, éventuellement après l'avoir converti au préalable en son chlorure d'acide, de manière à obtenir un composé de formule Ib:

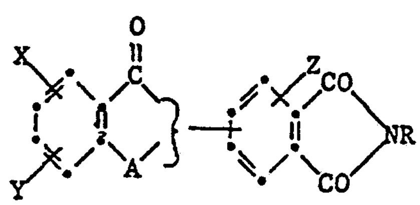

(Ib)

puis on transforme éventuellement celui-ci en un composé de formule I dans lequel E et E' représentent chacun, indépendamment l'un de l'autre, un radical -COOR", -CON(R")2 ou -CN ou forment ensemble un radical -CO-O-CO- ou un radical -CO-N(R)-CO dans lequel le symbole R peut avoir différentes significations.

9. Procédé pour préparer des composés de formule I selon la revendication 1 dans lesquels E et E' représentent chacun, indépendamment l'un de l'autre, un radical -COOR", -CON(R")2 ou -CN, procédé caractérisé en ce qu'on fait réagir un composé de formule IIa

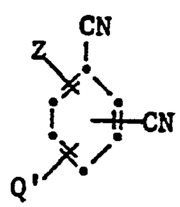

(IIa)

en présence d'une base, avec un composé de formule III ou avec un sel d'un composé de formule III, les réactions qui

donnent naissance à un composé de formule IVa:

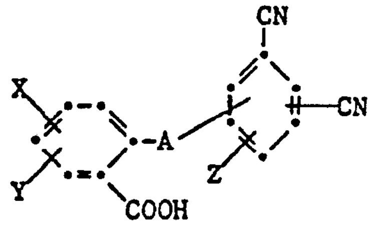

(IVa),

on cyclise le composé de formule IVa pour le convertir en un composé de formule Ic:

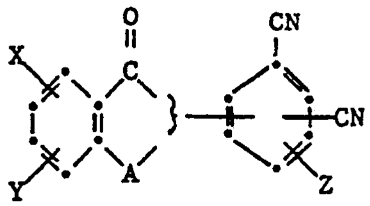

(Ic)

puis on transforme éventuellement celui-ci en un composé de formule I dans lequel E et E' représente chacun, indépendamment l'un de l'autre, un radical -COOR" ou -CON(R")2.

10. Procédé pour préparer des composés de formule I selon la revendication 1 dans lesquels E et E' se trouvent en position 1,3 et représentent chacun un radical -COOR" ou -CON(R")2, et Z représente l'hydrogène, procédé caractérisé en ce qu'on fait réagir un composé de formule IIb

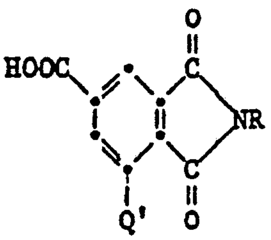

(IIb),

en présence d'une base, avec un composé de formule IIIa:

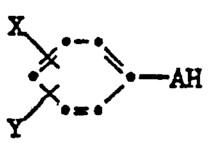

(IIIa)

ou avec un sel d'un tel composé, de manière à obtenir un composé de formule IVb:

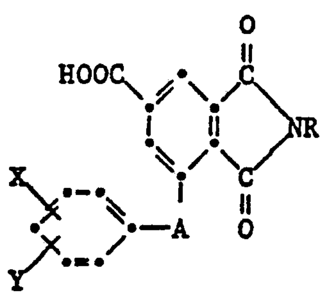

(IVb)

on transforme le composé de formule IVb, par hydrolyse, en l'acide dicarboxylique, on transforme cet acide dicarboxylique en son anhydride, on cyclise ce dernier en un composé de formule Id:

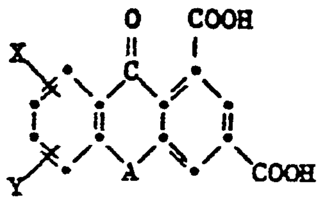

(Id)

puis on transforme éventuellement ce composé de formule Id en un composé de formule I dans lequel E et E' représentent chacun un radical -COOR" ou -CON(R")2, les symboles A, X et Y ayant les significations indiquées à la revendication 1 et Q' représentant un radical $-NO_2$, ou un atome d'halogène.

11. Application de composés de formule I selon la revendication 1 comme sensibilisateurs pour des polymères photoréticulables.

12. Application de composés de formule I selon la revendication 1 comme sensibilisateurs pour des matières photodurcissables, éventuellement colorées.